# EUROPEAN PATENT APPLICATION

(11) **EP 1 887 488 A1**
(43) Date of publication of application: **13.02.2008**
(21) Application number: 07252949.8
(22) Date of filing: 25.07.2007
(51) Int. Cl.: G06F 19/00

(54) **Vital information measuring device, managing device, and vital information communication system**

(30) Priority: 31.07.2006 JP 2006209147
(71) Applicant: Sharp Kabushiki Kaisha, Osaka-shi, Osaka 545-8522 (JP)
(72) Inventor: Nakagawa, Katsuya, Kyoto 619-0223 (JP); Niwamoto, Hiroaki, Chiba 266-0031 (JP)
(74) Representative: Brown, Kenneth Richard

(57) **Abstract**

A vital information measuring device measures vital information of a measurement target person. The vital information measuring device includes (i) a data frame creating section for generating, by using a symbolic code assigned to an item regarding measurement processing of the vital information, a request data frame for use in requesting a vital information managing device for necessary information, and (ii) a response data frame analyzing section for analyzing a response data frame, which is received from the vital information managing device. This makes it possible to provide a vital information measuring device that does not have a storage device having a particularly large storage space but can obtain precise vital information.

## Description

### FIELD OF THE INVENTION

The present invention relates to (i) a vital information measuring device, which measures vital information of a living body and establishes communication with a managing device for managing measurement processing information required in this process of measuring, (ii) the managing device, (iii) a vital information communication system, and the like.

### BACKGROUND OF THE INVENTION

In recent years, in view of increase of medical expenses and a coming aging society, good health maintenance increasingly has drawn people's attention and people have been expected to do health management in everyday life for themselves so as not to catch a disease. For example, for the health management, they control calorie intakes from meals and balance of nutrition in meals, and move their muscles through moderate physical exercises.

Meanwhile, as an instrument for supporting such health management, a measuring instrument such as a weight/body-fat scale, a thermometer, a blood pressure meter, or a passometer has been pervasive. In recent years, via communication established between each of these measuring instruments and an information processing device such as a personal computer, results of the measurement by the instruments are recorded onto and managed in the information processing device.

For example, Patent Citation 1 (Japanese Unexamined Patent Publication Tokukai 2005-333269 (published on December 2, 2005)) discloses a measurement data communication device, which establishes communication between such instruments and the information processing device and transmits, to the information processing device, measurement data obtained through the measurement carried out by each of the instruments.

Specifically, the measurement data communication device described in Patent Citation 1 includes (i) a measurement data generating section for generating measurement data in accordance with arbitrary code value and measurement value, and (ii) a communication information generating section for generating communication information in accordance with the code value and a measurement name. The measurement data communication device transmits the communication information to the information processing device (information acquiring device) before transmitting the measurement data thereto.

As such, before transmitting the measurement data to the information processing device, the measurement data communication device transmits thereto the correlation between (i) the measurement name which indicates the category of the measurement value and (ii) the code value corresponding to the measurement name. This allows the information processing device to recognize the measurement name of the measurement value, which has been transmitted as the measurement data.

However, with the above conventional configuration, it is impossible to obtain precise vital information.

Specifically speaking, vital information measuring devices such as a weight scale and a height scale do not have storage devices having large storage spaces, so that such vital information measuring devices cannot handle data having large size. In other words, in order to obtain the vital information more precisely, it is necessary to use results of measurements carried out in past, results of measurements carried out by other vital information measuring devices, and the like. However, in the conventional configuration, there is not provided a storage device that is capable of storing the results of the measurements carried out in past, the results of the measurements carried out by the other vital information measuring devices, and the like. Accordingly, these information cannot be used, so that precise vital information cannot be obtained.

If a storage device having a large storage space is provided in the conventional configuration, cost of manufacturing the vital information measuring device and the size of the vital information measuring device are increased.

### SUMMARY OF THE INVENTION

An object of the present invention is to provide (i) a vital information measuring device that has no storage device having a particularly large storage space but can obtain precise vital information; (ii) a managing device; and (iii) a vital information communication system.

To achieve the object, a vital information measuring device according to the present invention measures vital information of a living body and communicates with a managing device that manages measurement processing information necessary for measurement processing of the vital information, an identification code being assigned to an item regarding the measurement processing of the vital information, so as to specify the item, and the vital information measuring device includes: a request data generating section for generating, by using the identification code, request data for use in requesting the managing device for response data including the measurement processing data; and an analyzing section for analyzing the response data, which is received from the managing device in reply to the request data and is described by using the identification code.

Here, the wording "vital information" refers to, e.g., information that the measurement target person uses for his/her health management. Examples of the vital information include: height, weight or body fat, blood pressure, the number of steps in walk, body temperature, oxygen saturation in blood, and the like.

Examples of the measurement processing information include: (i) a measurement value measured by an external vital information measuring device, (ii) the age of the measurement target person, (iii) the sex of the measurement target person, and the like.

An example of the identification code is (i) a code value indicated by alphanumeric characters, (ii) a text code, or the like.

According to the above structure, the request data generating section is provided, so that it is possible to request the managing device for the measurement processing information required in the measurement processing of the vital information. Moreover, the analyzing section is provided, so that it is possible to analyze the response data received from the managing device and obtain the requested measurement processing information.

That is, the vital information measuring device according to the present invention is capable of making a request to the managing device for the measurement processing information required in the measurement processing, and capable of acquiring the measuring processing information. This makes it unnecessary for the vital information measuring device to include a storage device having a large storage space for the sake of storing the measurement processing information.

In this way, it is possible to obtain precise vital information, even though no storage device having a particular storage space is provided.

To achieve the object, a managing device according to the present invention provides, to a vital information measuring device that measures vital information of a living body, measurement processing information necessary for measurement processing carried out by the vital information measuring device, and the managing device includes: a measuring device correlation information storage device for storing measuring device correlation information indicating a correlation between an item regarding the measurement processing and an identification code, each of which is defined in the vital information measuring device; a data checking section for, when request data for requesting the measurement processing information is received from the vital information measuring device, checking the measurement processing information requested by the request data, with reference to the measuring device correlation information; a searching section for searching for the measurement processing information checked by the data checking section; and a response data generating section for generating, in accordance with the measuring device correlation information by using the identification code, response data including the measurement processing information searched by the searching section.

According to the above structure, the measuring device correlation information storage device is provided, so that it is possible to recognize the correlation between (i) the item regarding the measurement processing and (ii) the identification code, each of which is defined in the specific vital information measuring device. Moreover, the data checking section is provided, so that it is possible to check the measurement processing information requested by the vital information measuring device.

Further, the searching section and the response data generating section are provided, so that it is possible to appropriately search for the measurement processing information requested by the vital information measuring device, and to transmit the measurement processing information to the vital information measuring device as the response data.

Namely, in cases where the vital information measuring device makes a request for the measurement processing information, the managing device provides the vital information measuring device with the requested measurement processing information as the response data.

In this way, the measuring device according to the present invention provides the measurement processing information to the vital information measuring device, which does not have a storage device having a particularly large storage space, with the result that the vital information measuring device can obtain precise vital information.

To achieve the object, a vital information communication system according to the present invention includes: (1) a vital information measuring device, which measures vital information of a living body and communicates with a managing device that manages measurement processing information necessary for measurement processing of the vital information, an identification code being assigned to an item regarding the measurement processing of the vital information, so as to specify the item, said vital information measuring device including (i) a request data generating section for generating, by using the identification code, request data for use in requesting the managing device for response data including the measurement processing data and (ii) an analyzing section for analyzing the response data, which is received from the managing device in reply to the request data and is described by using the identification code; and (2) a managing device, which provides, to the vital information measuring device that measures the vital information of the living body, the measurement processing information necessary for the measurement processing carried out by the vital information measuring device, said managing device including (i) a measuring device correlation information storage device for storing measuring device correlation information indicating a correlation between the item regarding the measurement processing and the identification code, each of which is defined in the vital information measuring device, (ii) a data checking section for, when the request data for requesting the measurement processing information is received from the vital information measuring device, checking the measurement processing information requested by the request data, with reference to the measuring device correlation information, (iii) a searching section for searching for the measurement processing information checked by the data checking section, and (iv) a response data generating section for generating, in accordance with the measuring device correlation information by using the identification code, the response data including the measurement processing information searched by the searching section.

As such, in the vital information communication system, there is not provided a storage device having a particularly large storage space, but it is possible to obtain precise vital information.

To achieve the object, a method according to the present invention for controlling a vital information measuring device, which measures vital information of a living body and communicates with a managing device that manages measurement processing information necessary for measurement processing of the vital information, an identification code being assigned to an item regarding the measurement processing of the vital information, so as to specify the item, includes: a step of generating, by using the identification code, request data for use in requesting the managing device for response data including the measurement processing data; and a step of analyzing the response data, which is received from the managing device in reply to the request data and is described by using the identification code.

The above method includes the step of generating the request data, so that it is possible to make a request to the managing device for the measurement processing information required in the measurement processing of the vital information. Further, the above method includes the step of analyzing the response data, so that it is possible to analyze the response data sent from the managing device and obtain the requested measurement processing information.

That is, the method for controlling the vital information measuring device according to the present invention makes it possible to make a request to the managing device for the measurement processing information required in the measurement processing, and acquire the measuring processing information. This makes it unnecessary for the vital information measuring device to include a storage device having a large storage space for the sake of storing the measurement processing information.

In this way, it is possible to obtain precise vital information, even though no storage device having a particular storage space is provided.

To achieve the object, a method according to the present invention for controlling a managing device, which provides, to a vital information measuring device that measures vital information of a living body, measurement processing information necessary for measurement processing carried out by the vital information measuring device, includes: a step of storing measuring device correlation information indicating a correlation between an item regarding the measurement processing and an identification code, each of which is defined in the vital information measuring device; a step of, when request data for requesting the measurement processing information is received from the vital information measuring device, checking the measurement processing information requested by the request data, with reference to the measuring device correlation information; a step of searching for the measurement processing information checked by the data checking section; and a step of generating, in accordance with the measuring device correlation information by using the identification code, response data including the measurement processing information searched by the searching section.

The above method includes the step of storing the measuring device correlation information, so that it is possible to recognize the correlation between (i) the item regarding the measurement processing and (ii) the identification code, which are defined in the specific vital information measuring device. Moreover, the above method includes the step of checking the requested measurement processing information, so that it is possible to check the measurement processing information requested by the vital information measuring device.

Further, the above method includes the step of searching for the measurement processing information thus checked, and the step of generating the response data including the measurement processing information thus searched, so that it is possible to appropriately search for the measurement processing information requested by the vital information measuring device, and to transmit the measurement processing information to the vital information measuring device as the response data.

Namely, in cases where the vital information measuring device makes a request for the measurement processing information, the method for controlling the managing device makes it possible to provide the vital information measuring device with the requested measurement processing information as the response data.

In this way, the method for controlling the measuring device according to the present invention makes it possible to provide the measurement processing information to the vital information measuring device, which does not have a storage device having a particularly large storage space, with the result that the vital information measuring device can obtain precise vital information.

Additional objects, features, and strengths of the present invention will be made clear by the description below. Further, the advantages of the present invention will be evident from the following explanation with reference to the drawings.

### BRIEF DESCRIPTION OF THE DRAWINGS

Figure 1 deals with an embodiment of the present invention, and is a block diagram illustrating the configurations of main parts of a vital information measuring device.
Figure 2 deals with the embodiment of the present invention, and is a block diagram illustrating the configurations of main parts of a vital information communication system.
Figure 3(a) is a diagram illustrating one example of a device-instrument registration table according to the present embodiment.
Figure 3(b) is a diagram illustrating one example of an external device-instrument registration table according to the present embodiment.
Figure 3(c) is a diagram illustrating one example of an external device-instrument registration table according to the present embodiment.
Figure 4 deals with the embodiment of the present invention, and is a block diagram illustrating the configurations of main parts of a vital information managing device.
Figure 5 is a diagram illustrating one example of a user information table according to the present embodiment.
Figure 6 is a diagram illustrating one example of a vital information table according to the present embodiment.
Figure 7 is a diagram illustrating one example of a registered instrument table according to the present embodiment.
Figure 8 is a diagram illustrating one example of a user measurement information table according to the present embodiment.
Figure 9 is a diagram illustrating one example of the data structure of a data frame according to the present embodiment.
Figure 10 is a table illustrating one example of text data set as a communication identifier according to the present embodiment.
Figure 11 is a table illustrating one example of text data set as a service identifier according to the present embodiment.
Figure 12 is a diagram illustrating one example of the data structure of a data field according to the present embodiment.
Figure 13 is a flowchart illustrating one example of registration processing in the vital information communication system according to the present embodiment.
Figure 14 is a flowchart illustrating one example of check processing for a registered vital information measuring device in the vital information communication system according to the present embodiment.
Figure 15 is a flowchart illustrating one example of user information query processing in the vital information communication system according to the present embodiment.
Figure 16 deals with another embodiment of the present invention, and is a block diagram illustrating the configurations of main parts of a vital information managing device.

### DESCRIPTION OF THE EMBODIMENTS

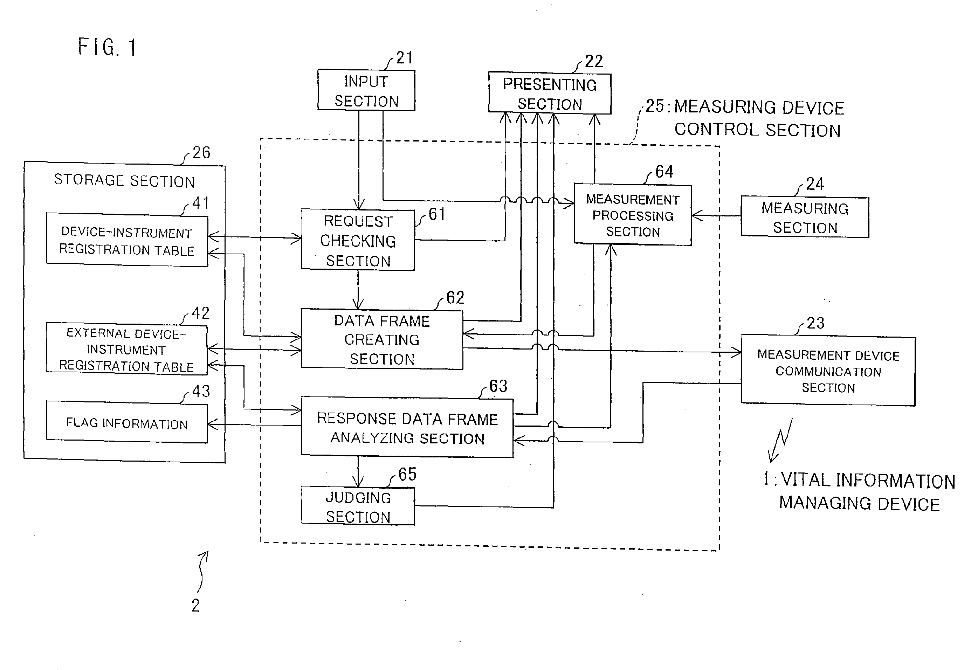

One embodiment of the present invention will be described below with reference to Figure 1 to Figure 16. Specifically, a vital information communication system 100 according to the present embodiment is a system in which measurement data of measurement carried out by a vital information measuring device 2 is recorded and managed in a vital information managing device (managing device) 1, and in which measurement data managed by the vital managing device 1 is provided to the vital information measuring device 2.

The vital information communication system 100 includes one or more vital information measuring devices 2 and the vital information managing device 1. Each of the vital information measuring devices 2, and the vital information managing device 1 are connected to each other such that they can communicate with each other.

The vital information measuring device 2 is a measuring instrument, which is used by a measurement target person (user) for the purpose of his/her health management. Examples of the vital information measuring device 2 include a weight scale, a height scale, a blood pressure meter, a pulse rate meter, a urine glucose meter, a blood glucose meter, a passometer, and the like.

For ease of explanation, the followings are assumed in the vital information communication system 100 according to the present embodiment. The vital information communication system 100 includes a vital information measuring device 2a, a vital information measuring device 2b, and a vital information measuring device 2c. The vital information measuring device 2a is a weight scale X provided by an X company, the vital information measuring device 2b is a height scale Y provided by a Y company, and the vital information measuring device 2c is a height scale Z provided by a Z company. The height scales Y and Z are height scales having different performances in measurement precision. Specifically, in the present embodiment, the height scale Z has a measurement precision higher than that of the height scale Y.

When the vital information measuring device 2a, the vital information measuring device 2b, and the vital information measuring device 2c do not need to be distinguished from one another, each of them will be merely referred to as "vital information measuring device 2".

The vital information measuring devices 2 provided in the vital information communication system 100 are not limited only to the vital information measuring devices 2a, 2b, and 2c. Other vital information measuring device 2 may be provided therein. In other words, one or more vital measuring devices 2 may be provided in the vital information communication system 100.

The vital information managing device 1 establishes communication with each of the vital information measuring devices 2, receives measurement data from the vital information measuring device 2, and stores the measurement data. In response to a request from the vital information measuring device 2, the vital information managing device 1 transmits thereto information requested by the vital information managing device 2.

The vital managing device 1 can be realized by, e.g., a personal computer, a mobile communication device, or the like, each of which has a function of managing information received from the vital information measuring device 2.

### (Configuration of vital information measuring device)

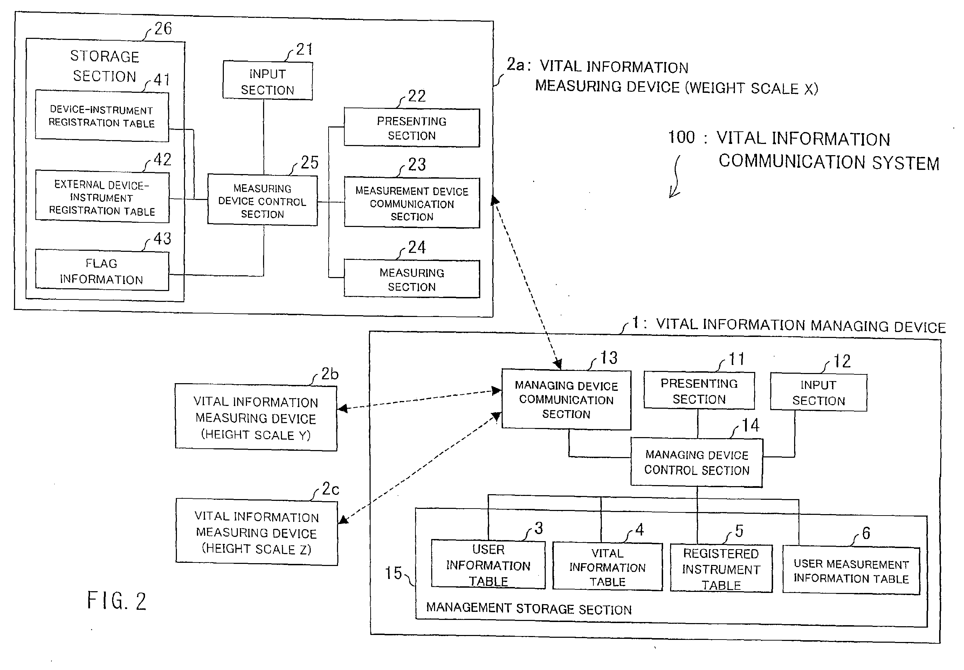

The following explains the configuration of each vital information measuring device 2 provided in the vital information communication system 100 according to the present embodiment, with reference to Figure 2. Figure 2 deals with this embodiment of the present invention and is a block diagram illustrating the configurations of main parts of the vital information communication system 100.

As shown in Figure 2, the vital information measuring device 2 includes an input section 21, a presenting section (measuring device output device) 22, a measuring device communication section 23, a measuring section 24, a measuring device control section 25, and a storage section (external device correlation information storage device) 26. Note that the configurations of the main parts of the vital information measuring devices 2a to 2c are the same, so that Figure 2 merely illustrates the configurations of the parts of the vital information measuring device 2a.

The presenting section 22 presents information, such as a measurement status or a measurement result, to a user. The presenting section 22 is made up of (i) a liquid crystal display section for displaying the information, (ii) an LED light emitting section, (iii) a speaker for outputting a sound effect, and the like. The presenting section 22 outputs the measurement status or the measurement result in response to an instruction from the measuring device control section 25 described later.

For example, in cases where the vital information measuring device 2 is the weight scale X (vital information measuring device 2a), the presenting section 22 outputs either (i) a measurement status in measuring a user's weight or (ii) a measurement result thereof. On the other hand, in cases where the vital information measuring device 2 is the height scale Y (vital information measuring device 2b) or the height scale Z (vital information measuring device 2c), the presenting section 22 outputs either (i) a measurement status in measuring the height of a user of the vital information communication system 100 or (ii) a measurement result thereof. Note that the wording "measurement status" herein refers to, e.g., information regarding measurement processing, such as information indicating whether or not measurement is being currently carried out, and information indicating whether or not the measurement has been finished. Further, when a device-instrument registration table 41 provided in the vital information measuring device 2 is registered in the vital information managing device 1, the presenting section 22 outputs a notification indicating that the registration has been done.

The measuring section 24 measures vital information of the user in response to a control instruction sent from the measuring device control section 25. For example, in cases where the vital information measuring device 2 is the weight scale X (vital information measuring device 2a), the measuring section 24 is realized by a weight sensor or a sensor for measuring an electric resistance of a living body. In response to an instruction from the measuring device control section 25, the measuring section 24 measures the user's weight or the user's body fat.

In cases where the vital information measuring device 2 is the height scale Y (vital information measuring device 2b) or the height scale Z (vital information measuring device 2c), the measuring section 24 is realized by a sensor for measuring a length. In response to an instruction from the measuring device control section 25, the measuring section 24 measures the user's height.

The input section 21 is used in instructing the vital information measuring device 2 to (i) start various operations on the vital information measuring device 2, (ii) register various information therein, and (iii) make queries thereto for the various information. The input section 21 may be provided with, e.g., a plurality of operational buttons corresponding to various instructions. The input section 21 may be operational keys, numeral keys, a touch panel, or the like, by each of which the instructions are executed.

The measuring device control section 25 controls various processing carried out by the respective sections provided in the vital information measuring device 2 according to the present embodiment. Details thereof will be described later.

The storage section 26 is a readable/writable storage medium, and can be realized by, e.g., a flash EEPROM, a hard disk, or the like. The storage section 26 stores (i) the device-instrument registration table 41 to be registered in the vital information managing device 1, (ii) external device-instrument registration tables (external device correlation information) 42, and (iii) flag information (existence information) 43.

The device-instrument registration table 41 is an instrument registration table indicating a correlation between (i) symbolic codes defined in the vital information measuring device 2 and (ii) information indicated by the symbolic codes. More specifically, what are correlated with one another and managed in the device-instrument registration table 41 are: (i) an instrument name (device information) for specifying the vital information measuring device 2, (ii) category names respectively indicating categories of information to be sent and received, (iii) unit information indicating a unit of information handled based on each category name, and (iv) the symbolic codes for respectively identifying the category names.

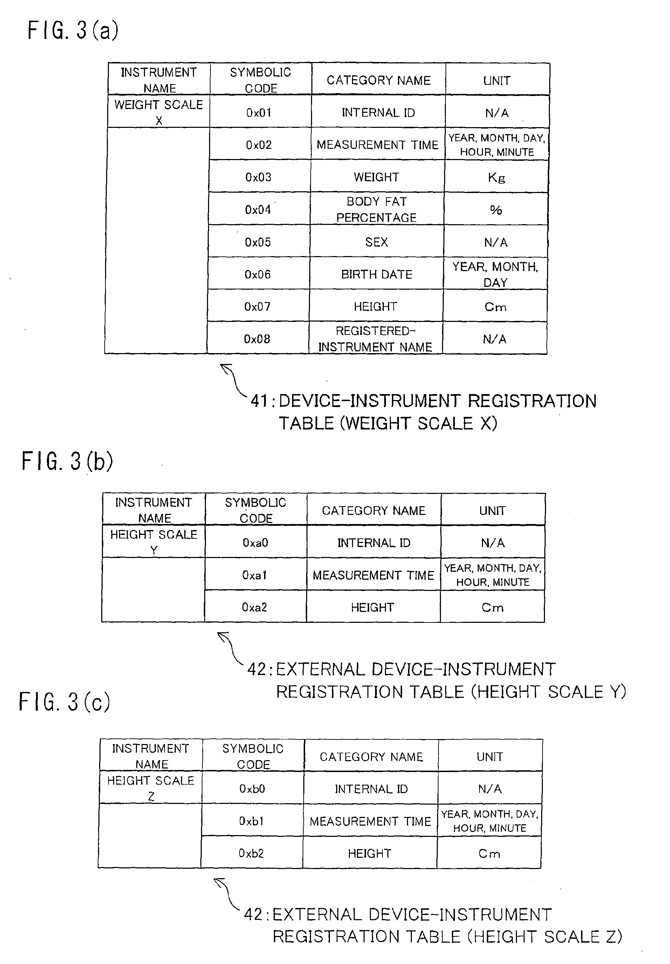

For example, in cases where the vital information measuring device 2 is the weight scale X (vital information measuring device 2a), the "weight scale X" is recorded as the instrument name in the device-instrument registration table 41 as shown in Figure 3(a). Further, there is a correlation among (i) the symbolic codes respectively identifying the categories of data to be exchanged between the weight scale X and the vital information managing device 1, (ii) the category names respectively indicating the contents of the categories, and (iii) the units respectively used for the values of the information handled based on the category names. Note that Figure 3(a) is a diagram illustrating one example of the device-instrument registration table 41 according to the present embodiment.

The external device-instrument registration tables 42 are instrument registration tables indicating correlations between (i) symbolic codes defined in the external vital information measuring devices 2 (vital information measuring device 2b and vital information measuring device 2c) and (ii) information indicated by the symbolic codes. The external device-instrument registration tables 42 are information used upon making queries to the vital information managing device for measurement values measured by the external vital information measuring devices 2. Specifically, with reference to each of the external device-instrument registration tables 42, the vital information measuring device 2 designates a symbolic code indicating the category of a measurement value or the like measured by an external vital information measuring device 2, thereby requesting the vital information managing device 1 for the measurement value or the like measured by the external vital information measuring device 2.

More specifically, as shown in Figure 3(b) and Figure 3(c), in the external device-instrument registration table 42 regarding the vital information measuring device 2b, the "height scale Y" is recorded as the instrument name. Further, there is a correlation among (i) the symbolic codes respectively identifying the categories of data to be exchanged between the height scale Y and the vital information managing device 1, (ii) the category names respectively indicating the contents of the categories, and (iii) the units respectively used for the values of the information handled based on the category names. In the meanwhile, in the external device-instrument registration table 42 regarding the vital information measuring device 2c, the "height scale Z" is recorded as the instrument name. Further, there is a correlation among (i) the symbolic codes respectively identifying the categories of data to be exchanged between the height scale Z and the vital information managing device 1, (ii) the category names respectively indicating the contents of the categories, and (iii) the units respectively used for the values of the information handled based on the category names. Note that each of Figure 3(b) and Figure 3(c) is a diagram illustrating one example of the external device-instrument registration table 42 according to the present embodiment.

As described above, in the present embodiment, the instrument registration tables provided in the vital information measuring device 2 are distinguished from each other as follows: the instrument registration table regarding the vital information measuring device 2 is the device-instrument registration table 41 whereas the instrument registration tables regarding the external vital information measuring devices 2 are the external device-instrument registration tables 42.

The flag information 43 is information indicating whether or not an instrument registration table regarding an external vital information measuring device 2 is registered in the vital information managing device 1. The vital information measuring device 2 is capable of making a query to the vital information managing device 1 about an external vital information measuring device 2 whose measurement value or the like has been registered in the vital information managing device 1. Details thereof will be described later. In cases where it is found as a result of this query that the vital information managing device 1 has the measurement value or the like of the target vital information measuring device 2, the vital information measuring device 2 turns on a flag indicating the target vital information measuring device 2.

### (Configuration of measuring device control section)

The following explains the configuration of the measuring device control section in detail with reference to Figure 1. Figure 1 deals with this embodiment according to the present invention and is a block diagram illustrating the configurations of the main parts of the vital information measuring device 2.

As shown in Figure 1, the measuring device control section 25 includes a request checking section 61, a data frame creating section (request data generating section) 62, a response data frame analyzing section (analyzing section) 63, and a measurement processing section (calculating section) 64, each of which serves as a function block of the measuring device control section 25. In cases where the measuring device control section 25 can be realized by, e.g., a CPU or the like, the CPU reads out a program from a ROM (not shown) to a RAM or the like and executes it, thereby realizing these sections of the measuring device control section 25.

The request checking section 61 checks a user's instruction sent from the input section 21. For example, in cases where the user makes an instruction of registering the device-instrument registration table 41 in the vital information managing device 1, the request checking section 61 reads out the device-instrument registration table 41. Thereafter, the request checking section 61 instructs the data frame creating section 62 to create a data frame (request data) to be transmitted to the vital information managing device 1. On this occasion, the request checking section 61 also instructs the presenting section 22 to output a notification indicating that the vital measuring device 2 is currently communicating with the vital information managing device 1.

Now, consider another example. In cases where the user makes an instruction of making a query to the vital information managing device 1 about a vital information measuring device 2 whose measurement value has been registered therein, the request checking section 61 instructs the below-described data frame creating section 62 to create a data frame for use in making this query. Further, in cases where the user makes an instruction of, e.g., measuring the user's body fat, the request checking section 61 checks user information required for the measurement of body fat percentage. Thereafter, the request checking section 61 instructs the below-described data frame creating section 62 to create a data frame for use in making a query for the required user information.

In response to such instructions from the request checking section 61, the data frame creating section 62 makes reference to the device-instrument registration table 41 so as to create the data frames for use in instructing various processing to be carried out with respect to the vital information managing device 1. Examples of the various processing include registration of information and request making. Then, the data frame creating section 62 controls the measuring device communication section 23 such that the measuring device communication section 23 transmits the created data frame to the vital information managing device 1. In the meanwhile, for example, in cases where an instruction of registering the device-instrument registration table 41 in the vital information managing device 1 is made, the data frame creating section 62 creates a data frame for use in notifying the instruction and causes the presenting section 22 to present that the device-instrument registration table 41 has been registered in the vital information managing device 1.

The response data frame analyzing section 63 analyzes a data frame received from the vital information managing device 1. Further, the response data frame analyzing section 63 analyzes a response data frame (response data) received in reply to a query to the vital information managing device 1 about an external vital information measuring device registered in the vital information managing device 1. In cases where it is found as a result of the analysis that a measurement value or the like of the target vital information measuring device 2 has been registered in the vital information managing device 1, the response data frame analyzing section 63 turns on a flag corresponding to the target vital information measuring device 2.

The measurement processing section 64 carries out calculation processing so as to find weight, height, body fat, or the like, in accordance with at least one of (i) a sensor output obtained by the measuring section 24 and (ii) information acquired from the vital information managing device 1. The measurement processing section 64 causes the presenting section 22 to output a result of the calculation, or transmits the result to the data frame creating section 62 and instructs the data frame creating section 62 to create a data frame for use in notifying the result to the vital information managing device 1.

### (Configuration of vital information managing device)

The following explains the configuration of the vital information managing device 1 with reference to Figure 2. As shown in Figure 2, the vital information managing device 1 includes a presenting section 11, an input section 12, a managing device communication section 13, a managing device control section 14, and a management storage section (measuring device correlation information storage device, measurement processing information storage device, measurement target person information storage device, measurement result information storage device) 15.

The presenting section 11 presents, to a user of the vital information communication system 100, information stored in the management storage section 15, in response to an instruction sent from the managing device control section 14. The present section 11 is made up of a liquid crystal display section for displaying the information, an LED light emitting section, a speaker for outputting a sound effect, and the like.

The input section 12 is input means for feeding information to the management storage section 15 and editing information stored therein. The input section 12 can be realized by, e.g. a keyboard, upward and downward keys, numeral keys, a mouse, or the like.

The managing device communication section 13 establishes communication between the vital information managing device 1 and the vital information measuring device 2, in response to an instruction from the managing device control section 14. The managing device communication section 13 exchanges, with the vital information managing device 2, a measurement result (measurement value), information necessary for measurement, and the like.

The managing device control section 14 carries out various types of control over the sections constituting the vital information managing device 1. Details thereof will be described later.

The management storage section 15 is a readable/writable storage medium, and can be realized by, e.g., a flash EEPROM, a hard disk, or the like. The management storage section 15 stores a user information table (measurement processing information, measurement target person information) 3, a vital information table (measurement processing information, measurement result information) 4, a registered instrument table (measuring device correlation information) 5, and a user measurement information table 6.

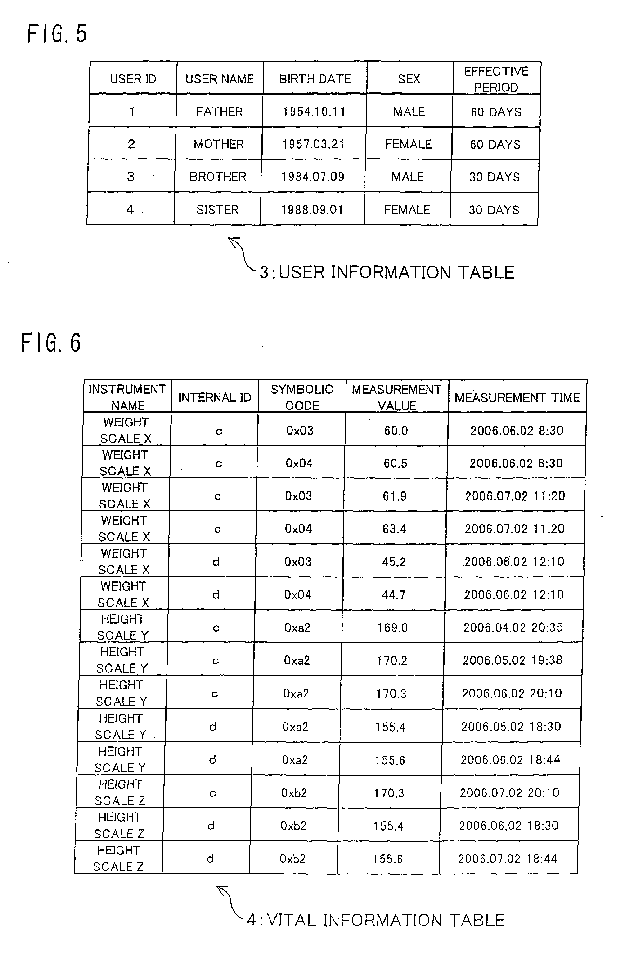

The user information table 3 includes information regarding a user. Specifically, as shown in Figure 5, the user information table 3 is a table storing user IDs, user names, birth dates, sexes, and effective periods such that they are correlated with one another. Each of the effective periods defines a period during which relevant vital information is effective. Note that Figure 5 is a diagram illustrating one example of the user information table 3 according to the present embodiment.

Here, the user IDs are identification information set in the vital information managing device 1 so as to specify the users, respectively.

Further, each of the effective periods is a period from the present moment to a past date until which a measurement result is determined to be effective, i.e., is a period during which the measurement result is guaranteed to be effective (reliable). In the present embodiment, the effective period is set in accordance with the age of a user who is to be subjected to measurement. For example, compare a user in early teens with a user in thirties. The height of the user in early teens changes greatly during a given period, whereas the height of the user in thirties hardly changes during the given period. If the effective period is the same between the user in early teens and the user in thirties, reliabilities of the measurement results thereof at the present moment differ.

When the vital information measuring device 2 requests supply of a measurement value but the measurement value is out of its effective period, the managing device control section 14 instructs the presenting section 11 to output a notification of suggesting the user remeasurement.

The vital information table 4 includes information regarding measurement results (measurement values) received from the vital information measuring devices 2. Specifically, as shown in Figure 6, the vital information table 4 is a table indicating a correlation among (i) instrument names respectively assigned to the vital information measuring devices 2, (ii) internal IDs defined in the vital information measuring devices 2, (iii) the symbolic codes defined in the vital information measuring devices 2, (iv) the measurement values, and (v) measurement times. Note that Figure 6 is a diagram illustrating one example of the vital information table 4 according to the present embodiment.

In cases where each of the vital information measuring devices 2 according to the present embodiment is shared by a plurality of users, the vital information measuring device 2 is configured such that a measurement result or information necessary for measurement can be managed for each user. In order that information can be managed based on users in the vital information measuring device 2, the internal IDs, which are identification information, are respectively assigned to the users.

The registered instrument table 5 is a table for storing and managing the device-instrument registration table 41 received from each of the vital information measuring devices 2. The registered instrument table 5 is information used for recognition of information indicated by the symbolic codes defined in the vital information measuring devices 2. In the present embodiment, each of the instrument registration tables, and the registered instrument table 5 are different from each other as follows. The instrument registration table stores the correlation between the category names and the symbolic codes in each of the vital information managing devices 2 as described above. On the other hand, the registered instrument table is managed by the vital information managing device 1, and is information in which the instrument registration table is correlated with (i) the name of the corresponding measuring device and (ii) the address thereof.

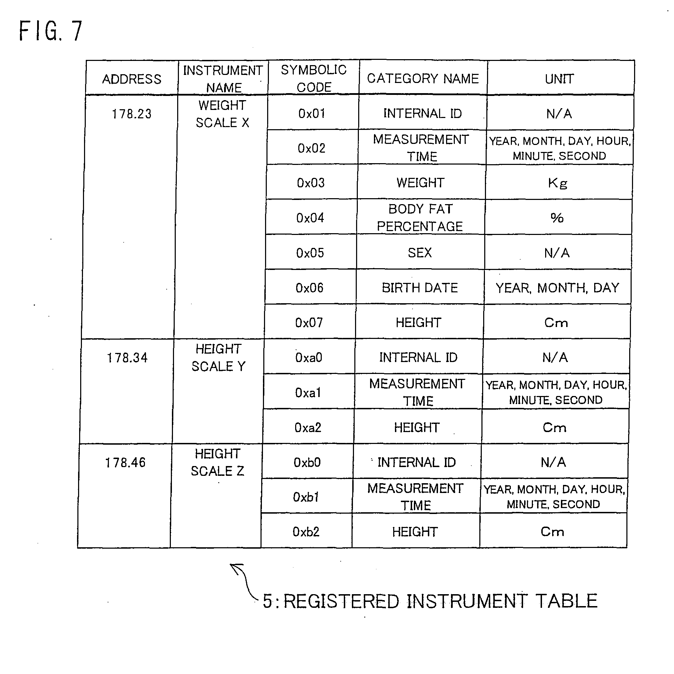

Specifically, see Figure 7. The registered instrument table 5 stores (i) the information represented by the device-instrument registration tables 41 which have been respectively transmitted from the vital measuring devices 2 and in which the instrument names, the symbolic codes, the category names, and the units are correlated with one another and (ii) the addresses for respectively specifying the vital measuring devices 2, in such a manner that the information represented by the device-instrument registration tables 41 and the addresses are correlated with each other. Note that Figure 7 is a diagram illustrating one example of the registered instrument table 5 according to the present embodiment.

Every time the vital information managing device 1 according to the present embodiment receives a device-instrument registration table 41 from the same address, the managing device control section 14 overwrites the registered instrument table 5. Meanwhile, when the vital information managing device 1 receives a device-instrument registration table 41 from a new transmitting end (unregistered address), the managing device control section 14 adds this received device-instrument registration table 41 in the registered instrument table 5 such that the device-instrument registration table 41 is correlated with this new address.

Note that: it is assumed that the vital information managing device 1 according to the present embodiment receives the device-instrument registration tables 41 from the weight scale X, the height scale Y, and the height scale Z, respectively, and has recorded them onto the registered instrument table 5.

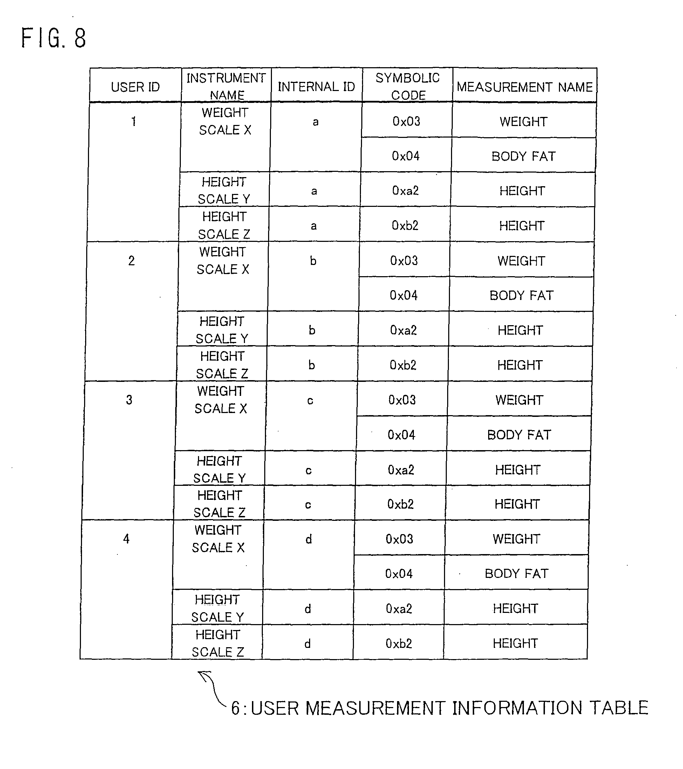

The user measurement information table 6 is information for use in searching for requested information in response to a query sent from each of the vital information measuring devices 2. As shown in Figure 8, the user measurement information table 6 indicates the user IDs, the instrument names of the devices used by the users, the internal IDs respectively set in the devices, measurement names indicating the categories of the measurements carried out by the devices, and the symbolic codes corresponding to the measurement names. Note that Figure 8 is a diagram illustrating one example of the user measurement information table 6 according to the present embodiment.

As described above, the vital information managing device 1 receives the device-instrument registration table 41 from each of the vital information measuring devices 2 with which communication is established, records it onto the registered instrument table 5, and manages the registered instrument table 5.

Further, the vital information managing device 1 stores the vital information table 4 in which the internal IDs for identifying the users in each of the vital information measuring devices 2 and the measurement results (measurement values and measurement times) are correlated with each other, so that it is possible for the vital information managing device 1 to manages, based on the users, the data of the measurements carried out by the plurality of vital information measuring devices 2, respectively.

Further, the vital information managing device 1 stores the user information table 3, which is information necessary for the measurement to be carried out by each of the vital information measuring devices 2, so that it is possible for the vital information managing device 1 to provide, in response to a query from the vital information measuring device 2, the necessary information regarding each user. Examples of such information necessary for the measurement include: the sex of the user, the birth date thereof, and the like.

### (Configuration of managing device control section)

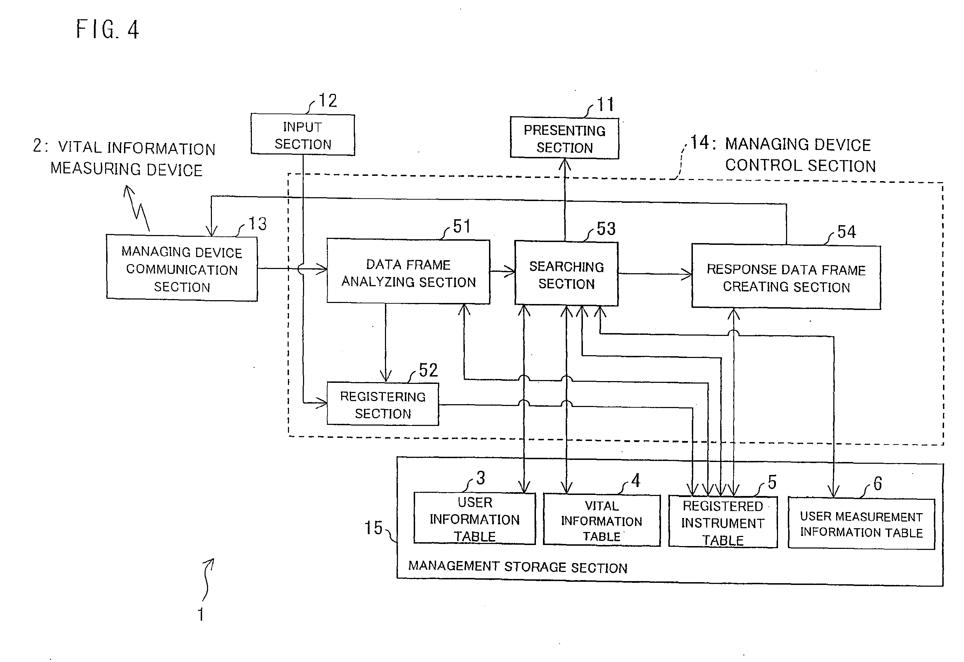

The following explains the configuration of the managing device control section 14 in detail with reference to Figure 4. Figure 4 deals with this embodiment according to the present invention and is a block diagram illustrating the configurations of main parts of the vital information managing device 1. As shown in Figure 4, the managing device control section 14 includes a data frame analyzing section (data checking section) 51, a registering section 52, a searching section 53, and a response data frame creating section 54, each of which serves as a function block of the managing device control section 14. In cases where the managing device control section 14 can be realized by, e.g., a CPU or the like, the CPU reads out a program from a ROM (not shown) to a RAM or the like and executes it, thereby realizing these sections of the managing device control section 14.

The data frame analyzing section 51 analyzes a data frame received from each of the vital information measuring devices 2. Specifically, the data frame analyzing section 51 reads out a service identifier from the received data frame and checks it. In cases where the data frame analyzing section 51 confirms that the service identifier is set to indicate "Registration", the data frame analyzing section 51 reads out, from the data frame, information regarding the device-instrument registration table 41, transmits the readout information to the below-described registering section 52, and instructs the registering section 52 to register the readout information.

On the other hand, in cases where the service identifier of the received data frame is set to indicate "Inquiry" and therefore represents a query about a registered vital information measuring device, the data frame analyzing section 51 instructs the searching section 53 to search for the registered vital information measuring device. For example, consider a case where the query is about user information regarding a user, such as a measurement value of the user's height, age, and sex. In this case, the data frame analyzing section 51 instructs the searching section 53 to search for the requested user information.

In response to the instruction from the data frame analyzing section 51, the registering section 52 records onto the management storage section 15 the information that is requested to be registered therein.

In response to the instruction from the data frame analyzing section 51, the searching section 53 searches for the requested information with reference to the user information table 3, the vital information table 4, the registered instrument table 5, and the user measurement information table 6, and the like. The searching section 53 sends a search result to the response data frame creating section 54, and instructs the response data frame creating section 54 to create a data frame to be transmitted to the vital information measuring device 2.

In response to the searching section 53, the response data frame creating section 54 creates the response data frame (response data), which includes the information requested to be transmitted to the vital information measuring device 2. When the data frame is created, the response data frame creating section 54 controls the managing device communication section 13 such that the managing device communication section 13 transmits the data frame to the vital information measuring device 2.

### (Structure of data frame)

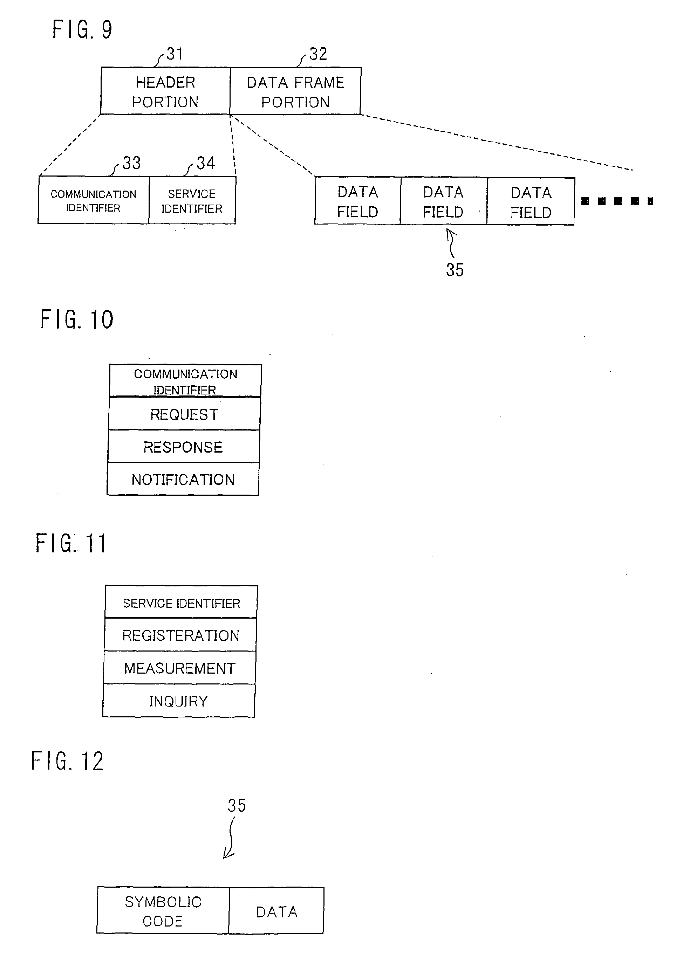

The following explains the frame structure of the information (data) to be exchanged between the vital information measuring device 2 and the vital information managing device 1, with reference to Figure 9 to Figure 12.

In the vital information communication system 100 according to the present embodiment, information (data) is exchanged between the vital information measuring device 2 and the vital managing device 1 in units of frame. A frame is made up of a header portion 31 and a data frame portion 32 as shown in Figure 9. Note that Figure 9 is a diagram illustrating one example of the data structure of the data frame according to the present embodiment.

In the header portion 31, a purpose of the data to be transmitted is described. The header portion 31 includes a communication identifier 33 and a service identifier 34. For example, in the vital information communication system 100 according to the present embodiment, the communication identifier 33 can be set to indicate any one of "Request", "Response", and "Notification" as shown in Figure 10. Figure 10 is a table showing one example of text data set as the communication identifier 33 according to the present embodiment.

For example, "Request" is to make a request to a transmission destination for certain information. Therefore, in the case of transmitting information making a certain request, the communication identifier 33 of the data frame including the information is set to indicate "Request".

"Response" is to make a response to such a communication identifier 33 indicating "Request" and contained in received information. Therefore, when making a response to such a data frame including the communication identifier 33 indicating "Request", the communication identifier 33 of the data frame to be transmitted in reply is set to indicate "Response".

"Notification" is to notify certain information to a transmission destination. For example, when the vital information measuring device 2 notifies either the device-instrument registration table 41 or a measurement value to the vital information managing device 1, the communication identifier 33 of the data frame including the information to be notified thereto is set to indicate "Notification".

In the meanwhile, the service identifier 34 is set to indicate any one of "Registration", "Measurement", and "Inquiry" as shown in Figure 11. The service identifier 34 is set to indicate "Registration", in the case of transmitting information requesting registration. Figure 11 is a table showing one example of text data set as the service identifier 34 according to the present embodiment.

For example, in the case of registering the device-instrument registration table 41 of the vital information measuring device 2 in the vital information managing device 1, the service identifier 34 of the data frame that includes the device-instrument registration table 41 and that is to be transmitted is set to indicate "Registration". The service identifier 34 is set to indicate "Measurement", in the case of transmitting information regarding a measurement value. The service identifier 34 is set to indicate "Inquiry", in the case of making an inquiry regarding certain information.

The data frame portion 32 is made up of a series of data fields 35 as shown in Figure 9. A data field is a unit of data. Each of the data fields 35 is made up of a combination of a symbolic code and data corresponding to the symbolic code, as shown in Figure 12. Note that Figure 12 is a diagram illustrating one example of the data structure of the data field 35 according to the present embodiment.

For example, when the service identifier 34 indicates "Measurement", the measurement value corresponding to the measurement name indicated by the symbolic code is contained in the data included in the data field 35.

Meanwhile, when the service identifier 34 indicates "Inquiry", only the measurement name indicated by the symbolic code is indicated in the data included in the data field 35 and the measurement value corresponding to the measurement name remains empty, thus making a query about the measurement value. When the vital information managing device 1 according to the present embodiment receives such a data frame that has the service identifier 34 indicating "Inquiry" and the data field 35 in which the data is empty, the vital information managing device 1 is set to put, in this empty data, a data value relevant to the query and transmit a response in reply to the inquiry.

When the service identifier 34 indicates "Registration", the data field 35 is described in a predetermined format, and the data frame including such a data field 35 is transmitted to the destination to which registration is to be carried out.

### (Processing flows in vital information measuring device)

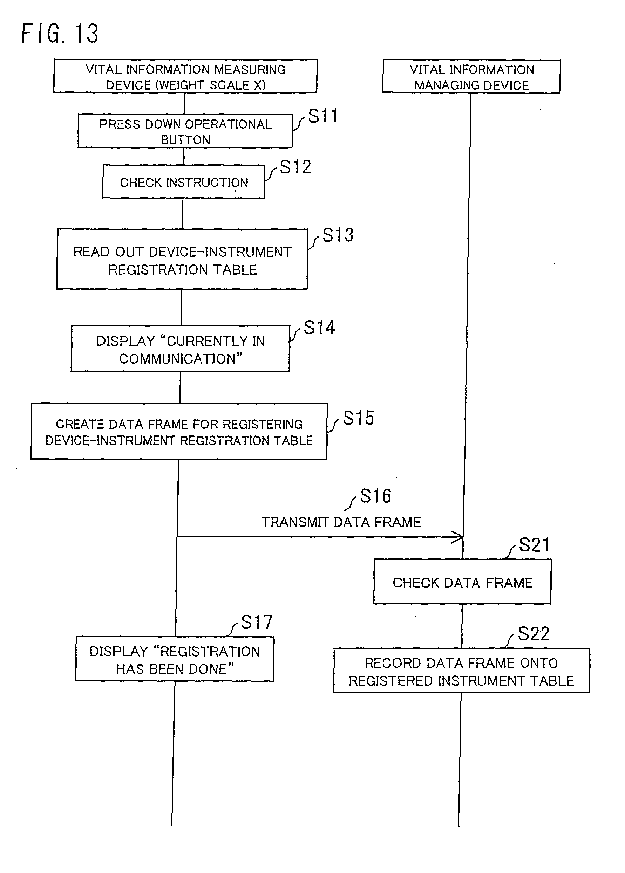

The following explains processing flows in the vital information measuring device 2, with reference to Figure 13 to Figure 15.

For ease of explanation, in the processing flows, it is assumed that the vital information measuring device 2 establishing communication with the vital information managing device 1 is the weight scale X (vital information measuring device 2a).

### (Registration processing)

Explained first is the registration processing for registering, in the vital information managing device 1, the device-instrument registration table 41 provided in the vital information measuring device 2a. The explanation will be made with reference to Figure 13, which is a flowchart illustrating one example of the registration processing in the vital information communication system 100 according to the present embodiment.

For exchange of data frames between the vital information managing device 1 and the vital information measuring device 2a, it is necessary that the information representing the device-instrument registration table 41 provided in the vital information measuring device 2a is transmitted to the vital information managing device 1 so as to be recorded onto the registered instrument table 5. A reason of this is as follows. That is, it is necessary to cause the vital information managing device 1 to recognize the correlation between (i) the symbolic codes defined in the vital information measuring device 2a and (ii) the categories respectively indicated by the symbolic codes.

In this way, the device-instrument registration table 41 of the vital information measuring device 2a is registered in the vital information managing device 1 in advance. This allows the vital information measuring device 2a and the vital information managing device 1 to use the symbolic codes in making a query for necessary measurement data and transmitting the necessary measurement data.

Now, this is concretely explained. When a user puts an operational button of the input section 21 of the vital information measuring device (weight scale X) 2a so as to instruct the vital information measuring device (weight scale X) 2a to register the device-instrument registration table 41 thereof in the vital information managing device 1 (Step S11; hereinafter, abbreviated as "S11").

When the user's instruction is inputted via the input section 21 as such, the request checking section 61 of the measuring device control section 25 of the vital information measuring device 2a checks the instruction (S12). In cases where the request checking section 61 confirms that the user's input is an instruction of registering the device-instrument registration table 41 in the vital information managing device 1, the request checking section 61 reads out the device-instrument registration table 41 from the storage section 26 (S13). Then, the request checking section 61 instructs the presenting section 22 to display, on the liquid crystal display section (not shown) of the presenting section 22, that the vital information measuring device 2a is currently communicating with the vital information managing device 1. In response to such an instruction from the request checking section 61 of the measuring device control section 25, the presenting section 22 displays "Currently in communication" (S14).

Next, in the measuring device control section 25, the request checking section 61 instructs the data frame creating section 62 to create a data frame to be used for the registration of the device-instrument registration table 41 in the vital information managing device 1. In response to such an instruction from the request checking section 61, the data frame creating section 62 creates the data frame as follows (S15).

That is, the data frame creating section 62 sets, in the data frame portion of the data frame to be transmitted, the content of the device-instrument registration table 41 read out from the storage section 26, sets the communication identifier of the header portion at "Notification", and sets the service identifier at "Registration".

After the data frame is created in this way, the data frame creating section 62 instructs the measuring device communication section 23 to transmit the data frame to the vital information managing device 1 so as to register the data frame therein (S16).

Further, after the data frame created as described above is transmitted to the vital information managing device 1, the data frame creating section 62 causes the presenting section 22 to present that the device-instrument registration table 41 has been registered in the vital information managing device 1 (S17).

When the vital information managing device 1 receives the data frame via the managing device communication section 13, the data frame analyzing section 51 analyzes the data frame so as to check the information contained in the data frame (S21).

Specifically, the data frame analyzing section 51 reads out the service identifier 34 from the received data frame and checks it. In cases where the data frame analyzing section 51 confirms that the service identifier 35 indicates "Registration", the data frame analyzing section 51 reads out, from the data frame portion 32, the information regarding the device-instrument registration table 41, transmits the readout information to the registering section 52, and instructs the registering section 52 to register the readout information.

In response to the instruction from the data frame analyzing section 51, the registering section 52 correlates (i) the received information regarding the device-instrument registration table 41 with (ii) the address of the transmitting end, i.e., the vital information measuring device 2a, and records the correlated information onto the registered instrument table 5 (S22).

In this way, the device-instrument registration table 41 provided in the vital information measuring device 2a is transmitted to the vital information managing device 1 and is registered therein.

As such, the vital information measuring device 2a according to the present embodiment is allowed to register the device-instrument registration table 41 thereof in the vital information managing device 1. This makes it possible that, after the registration, the vital information measuring device 2a transmits to the vital information managing device 1 various information indicated by the symbolic codes, and receives therefrom information described by the symbolic codes. In other words, the various information exchanged with the vital information managing device 1 can use the symbolic codes defined in the vital information measuring device 2a.

### (Check processing for registered vital measuring device)

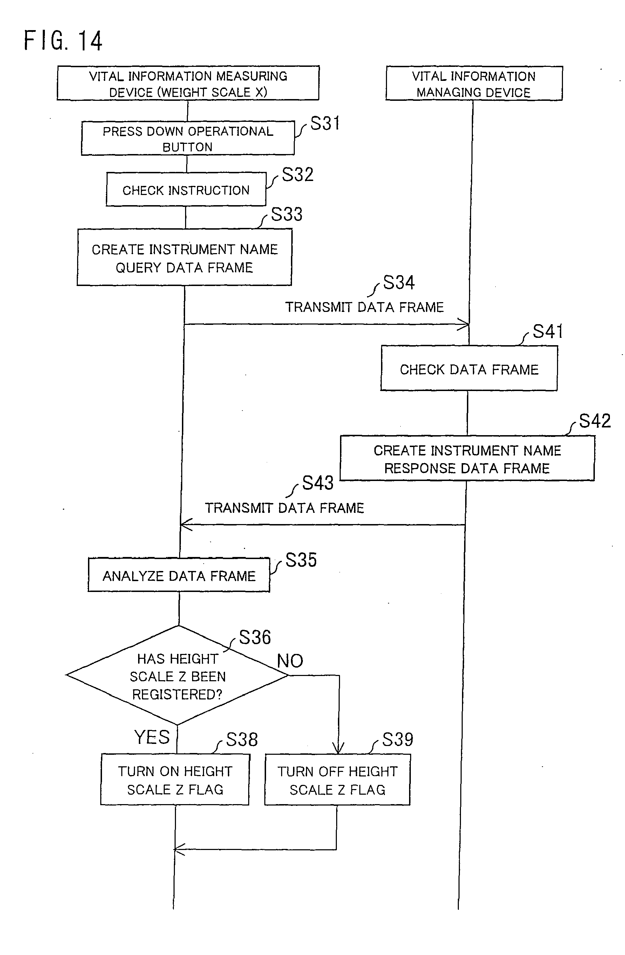

Explained next is processing (check processing for a registered vital measuring device) of checking whether or not a measurement value measured by an external vital information measuring device 2 (height scale Y or height scale Z) has been registered in the vital information managing device 1. The explanation will be made with reference to Figure 14, which is a flowchart illustrating one example of the check processing for a registered vital measuring device in the vital information communication system 100 according to the present embodiment.

For ease of explanation, it is assumed in the present embodiment that: the vital information measuring device (weight scale X) 2a makes a query to the vital information managing device 1 about vital information managing devices 2 whose measurement values have been registered therein, and the vital information measuring device 2a judges, in accordance with a result of the query, whether or not the measurement value of the height scale Z (vital information measuring device 2c) has been registered in the vital information managing device 1.

First, a user presses down an operational button of the input section 21 of the vital information measuring device 2a (weight scale X) so as to instruct the vital information managing device 1 to make a query to the vital information managing device 1 about vital information measuring devices 2 whose measurement values have been registered therein (S31).

When the user's instruction is inputted via the input section 21 as such, the request checking section 61 of the measuring device control section 25 of the vital information measuring device 2a checks the instruction (S32). In cases where the request checking section 61 confirms that the user's input is an instruction of making a query about the vital information measuring devices 2 whose measurement values have been already registered, the request checking section 61 instructs the data frame creating section 62 to create a data frame for use in making the query.

In response to such an instruction from the request checking section 61, the data frame creating section 62 sets the communication identifier 33 of the header portion 31 at "Request" and sets the service identifier 34 at "Inquiry" for the sake of making the above query. Then, the frame creating section 62 puts, in the data field 35, a symbolic code "0x08" corresponding to "registered instrument name", so as to create the data frame. In this way, the data frame creating section 62 creates the data frame (instrument name query data frame) for use in making the query about the vital information measuring devices 2 whose measurement values have been already registered (S33).

After the creation of the instrument name query data frame, the data frame creating section 62 controls the measuring device communication section 23 such that the created data frame is transmitted to the vital information managing device 1 (S34).

When the vital managing device 1 receives the instrument name query data frame from the vital information measuring device 2, the data frame analyzing section 51 makes reference to a part of the registered instrument table 5, which part corresponds to the vital information measuring device 2 whose address coincides with the address of the transmitting end having transmitted the data frame. With reference thereto, the data frame analyzing section 51 analyzes the information stored in the received data frame and described by the symbolic code, and checks the information (S41).

Here, think that the data frame analyzing section 51 reads out the service identifier 34 from the received data frame and confirms that the service identifier 34 is set to indicate "Inquiry". In this case, the data frame analyzing section 51 instructs the searching section 53 to search for the vital information measuring devices 2 whose measurement values have been already registered in the vital information managing device 1. In response to such an instruction from the data frame analyzing section 51, the searching section 53 makes reference to the registered instrument table 5 so as to search for the vital information measuring devices 2 whose measurement values have been already registered in the vital information managing device 1. Thereafter, the searching section 53 transmits a search result to the response data frame creating section 54, and instructs the response data frame creating section 54 to notify the search result to the vital information measuring device 2a having made the query.

In response to such an instruction from the searching section 53, the response data frame creating section 54 creates a response data frame (instrument name response data frame) replying to the query (S42). More specifically, the response data frame creating section 54 creates such an instrument name response data frame that includes the following contents.

That is, in the present embodiment, as shown in Figure 7, the registered instrument table 5 provided in the vital information managing device 1 stores the weight scale X, the height scale Y, and the height scale Z (vital information measuring devices 2a to 2c), so that the vital information managing device 1 creates such a data frame as to notify the instrument names of these three devices to the vital information measuring device (weight scale X) 2a. The data frame thus created serves as the instrument name response data frame replying to the query made from the vital information measuring device (weight scale X) 2a.

Namely, the response data frame creating section 54 sets the communication identifier 33 of the header portion 31 of the data frame at "Response", and sets the service identifier 34 thereof at "Inquiry". Set in the data frame portion 32 are: (i) a data field 35 in which a symbolic code is "0x08" and data indicates "weight scale X", (ii) a data field 35 in which a symbolic code is "0x08" and data indicates "height scale Y", and (iii) a data field 35 in which a symbolic code is "0x08" and data indicates "height scale Z".

When the response data frame creating section 54 creates the data frame as such in the vital information managing device 1, the managing device communication section 13 is so controlled as to transmit it to the vital information measuring device 2a (S43).

When the vital information measuring device 2a receives the instrument name response data frame from the vital information managing device 1, the response data frame analyzing section 63 of the vital information measuring device 2a analyzes this data frame (S35) so as to check the instrument names respectively stored in the data fields 35. The response data frame analyzing section 63 judges whether or not the measurement value of the vital information measuring device 2c (height scale Z) has been already registered in the vital information managing device 1 (S36).

In cases where the response data frame analyzing section 63 judges that the measurement value of the vital information measuring device 2c (height scale Z) has been already registered in the vital information managing device 1 ("YES" in S36), the response data frame analyzing section 63 turns on a flag corresponding to the height scale Z, and stores such flag information 43 in the storage section 26 (S37).

On the other hand, in cases where the response data frame analyzing section 63 judges "NO" in Step S36, the flag corresponding to the height scale Z is turned off and stores such flag information 43 in the storage section 26 (S38).

As described above, the vital information measuring device 2a according to the present embodiment is capable of having, as the flag information 43, a record of the external vital information measuring device 2c whose measurement value has been already registered in the vital information managing device 1. This allows the vital information measuring device 2a to recognize existence of the external vital information measuring device 2c, which is usable as a source of information.

Further, as described above, the vital information measuring device 2a according to the present embodiment is so configured as to manage, by using the flag information, the external vital information measuring device (height scale Z) 2c, which is usable as a source of information.

Here, in cases where the vital information measuring device 2a is not provided with the instrument registration table (external device-instrument registration table 42) regarding the vital information measuring device 2c, it is preferable to configure the vital measuring device 2a such that the instrument registration table is also received from the vital information managing device 1 in the aforementioned "check processing for a registered vital information measuring device".

In other words, in order to use the measurement result (measurement value) obtained by the vital information measuring device 2c, it is preferable that the instrument registration table regarding the external vital information measuring device 2c be contained in the instrument response data frame to be supplied from the vital information managing device 1 in Step S43.

### (User information query processing)

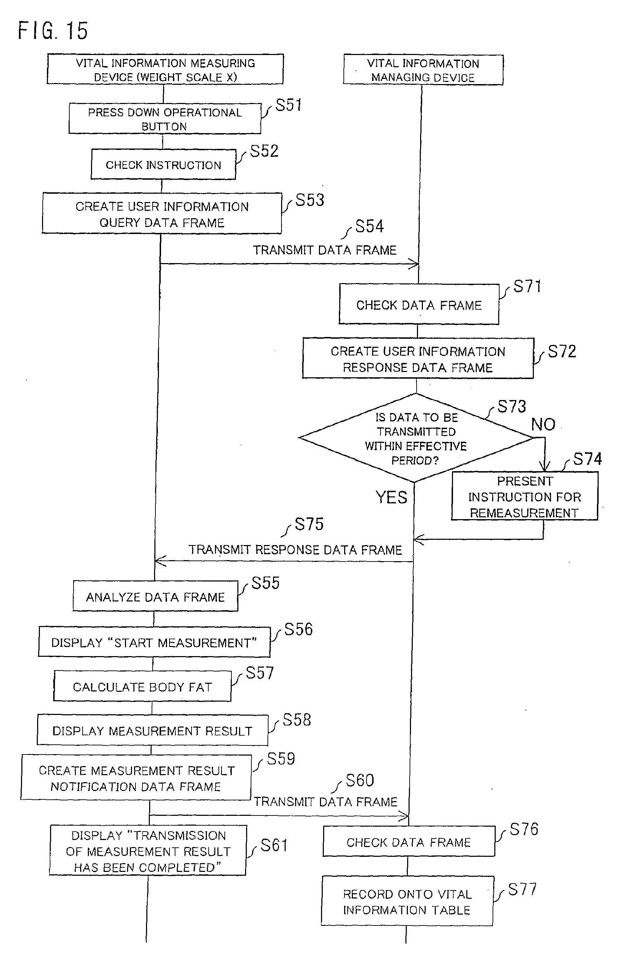

The following exemplifies processing of measuring a specific user's fat body percentage by using the vital information measuring device (weight scale X) 2a, so as to explain processing (user information query processing) of making a query to the vital information managing device 1 about user information. The explanation will be made with reference to Figure 15, which is a flowchart illustrating one example of the user information query processing in the vital information communication system 100 according to the present embodiment.

First, the vital information measuring device (weight scale X) 2a is shared by the plurality of users as described above. The weight scale X is configured to be capable of assigning the users the identifiers (internal IDs).

Now, assume that "d" is assigned to this specific user as the internal ID in this explanation for the "user information query processing".

First, the specific user presses down an operational button of the input section 21 of the vital information measuring device (weight scale X) 2a (S51) so as to indicate the user's internal ID and so as to instruct measurement of body fat percentage.

In response to the press-down of the operational button, the request checking section 61 of the measuring device control section 25 of the vital information measuring device 2a checks the instruction received from the user (S52). In cases where the request checking section 61 confirms that the instruction received from the user is to instruct the measurement of the user's body fat, the request checking section 61 checks for the user information necessary for the measurement of the user's body fat percentage. In other words, the vital information measuring device (weight scale X) according to the present embodiment does not have any information necessary for the body fat percentage measurement, such as the user's height, birth date, and sex. Especially, the vital information measuring device according to the present embodiment is configured to acquire the measurement value of the user's height, measured by the vital information measuring device 2b or 2c, as required and to use the measurement value thus acquired.

Therefore, the request checking section 61 specifies these necessary information in response to the user's instruction of measuring the body fat, and instructs the data frame creating section 62 to create a data frame (user information query data frame) for use in making a query for these information.

In response to the instruction from the request checking section 61, the data frame creating section 62 creates the user information query data frame for use in making the query to the vital information managing device 1 (S53).

Here, as described above, it is assumed in the present embodiment that the user's internal ID is "d" and the vital information measuring device 2a asks the vital information managing device 1 for the user's sex, birth date, and height. It is also assumed that the flag information in the storage section 26 indicates that the measurement value of the user's height measured by the height scale Z can be acquired and the vital information measuring device 2a therefore asks for the measurement value measured by the height scale Z.

The data frame creating section 62 of the measuring device control section 25 creates the user information query data frame in the following manner.

Specifically, the data frame creating section 62 sets the data user information data frame such that the communication identifier 33 of the header portion 31 thereof indicates "Request" and the service identifier 34 of the header portion 31 thereof indicates "Inquiry". The header portion 31 is thus set, with the result that the data frame to be transmitted from the vital information measuring device 2a to the vital information managing device 1 is set to indicate that the data frame requests inquiry of certain information.

Thereafter, the data frame creating section 62 puts, in the first data field 35 of the data frame portion 32, the symbolic code "0x01" and its corresponding data "d". The symbolic code "0x01" is a code indicating the internal ID, so that it is possible to indicate, in accordance with the symbolic code and the data, that the internal ID is "d".

Further, the data frame creating section 62 puts the symbolic code "0x05" but leaves its corresponding data "empty", in the second data field 35. The symbolic code "0x05" indicates sex, but the data corresponding to the symbolic code is "empty", thus indicating that information about sex is being requested.

Further, the data frame creating section 62 puts the symbolic code "0x06" but leaves its corresponding data "empty", in the third data field 35. The symbolic code "0x06" indicates birth date, but the data corresponding to the symbolic code is "empty", thus indicating that the information about birth date is being requested.

Further, the data frame creating section 62 puts the symbolic code "0x08" and its corresponding data "height scale Z" in the fourth data field 35. Specifically, the symbolic code "0x08" indicates the name of a registered instrument whose measurement value has been already registered, and the data corresponding to the symbolic code is "height scale Z", thus indicating that the information that the height scale Z has is being requested.

Further, the data frame creating section 62 puts the symbolic code "0xb2" but leaves its corresponding data "empty", in the fifth data field 35. The symbolic code "0xb2" indicates the "height" defined in the height scale Z, but the data corresponding to the symbolic code is "empty", thus indicating that the measurement value of the height measured by the height scale Z is being requested.

The data frame creating section 62 creates the user information query data frame in this way, and then controls the measuring device communication section 23 such that the measuring device communication section 23 transmits the user information query data frame to the vital information managing device 1 (S54).

When the vital managing device 1 receives the user information query data frame from the vital information measuring device 2a, the data frame analyzing section 51 makes reference to a part of the registered instrument table 5, which part corresponds to the vital information measuring device 2a whose address coincides with the address of the transmitting end having transmitted the data frame. With reference thereto, the data frame analyzing section 51 analyzes the information stored in the received data frame and described by the symbolic code, and checks the information (S71).

Here, because the address of the transmitting end having transmitted the data frame coincides with that of the vital information measuring device (weight scale X) 2a, so that the data frame analyzing section 51 makes reference to a part, indicating the symbolic codes defined in the vital information measuring device (weight scale X) 2a, of the registered instrument table 5. Then, the data frame analyzing section 51 interprets the information contained in the data fields 35.

More specifically, in accordance with the information contained in the first data field 35, the data frame analyzing section 51 recognizes that the query regards the user whose internal ID is "d". Further, the information stored in the second data field 35 indicates the query about sex, and the information stored in the third data field 35 indicates the query about birth date. Further, the information stored in the fourth data field 35 indicates that the information stored in the data fields 35 coming after the fourth data field 35 is defined by the symbolic codes defined in the height scale Z. In accordance with the fifth data field 35, the data frame analyzing section 51 recognizes that the query is about the value of the height measured by the height scale Z.

When the data frame analyzing section 51 thus recognizes each of the information requested by the received data frame, the data frame analyzing section 51 instructs the searching section 53 to search for each of the information. Here, from the user measurement information table 6 shown in Figure 8, it is found that the internal ID "d" of the vital information measuring device (weight scale X) 2a corresponds to the user ID "4" assigned in the vital information managing device 1. The searching section 53 searches for the sex and birth date of the user whose user ID is "4", in the user information table 3 shown in Figure 5. As a result of the search, it is recognized that the sex of the user whose user ID is "4" is "female" and her birth date is "September 1, 1988". Further, the searching section 53 makes reference to the vital information table 4 shown in Figure 6 and finds that there are recorded the following two measurement values (symbolic code 0xb2) measured by the height scale Z and regarding the user corresponding to the internal ID "d" in the weight scale X: (1) a measurement value at 18:30 on June 2, 2006, and (2) a measurement value at 18:44 on July 2, 2006.

Here, the user information query data frame received from the vital information measuring device (weight scale X) 2a according to the present embodiment does not designate any measurement time regarding measurement value. In the case where any specific measurement value is not designated as such, the vital information managing device 1 selects a measurement value measured most recently, and transmits it to the vital information measuring device 2a.

As described above, when the vital information managing device 1 receives the user information query data frame from the vital information measuring device (weight scale X) 2a, the searching section 53 searches for the information requested by the data frame. The searching section 53 transmits, to the response data frame creating section 54, the information found as a result of the search, and instructs the response data frame creating section 54 to create a response data frame. In response to such an instruction from the response data frame creating section 54, the response data frame creating section 54 creates the response data frame (S72).

More specifically, with reference to the registered instrument table 5, the response data frame creating section 54 creates the response data frame to be transmitted, in the following manner. That is, the request data frame creating section 54 sets the communication identifier 33 of the header portion 31 of the data frame at "Response" and the service identifier 34 thereof at "Inquiry". With these settings, the response data frame is allowed to indicate that the response data frame serves as a response to the inquiry.

Further, the response data frame creating section 54 sets, in the first data field 35 of the data frame portion 32, (i) "0x01" as the symbolic code defined in the vital information measuring device 2a to which the response data frame is to be transmitted, and (ii) "d" as the data corresponding to the symbolic code. The first data field 35 thus set indicates that the data is about the user whose internal ID in the weight scale X is "d".

The response data frame creating section 54 sets, in the second data field 35, (i) "0x05" as the symbolic code, and (ii) "female" as the data corresponding to the symbolic code. The second data field 35 thus set indicates that the sex of the user whose internal ID in the weight scale X is "d" is "female".

The response data frame creating section 54 sets, in the third data field 35, (i) "0x06" as the symbolic code, and (ii) "September 1, 1988" as the data corresponding to the symbolic code. The third data field 35 thus set indicates that the birth date of the user whose internal ID in the weight scale X is "d" is "September 1, 1988".

The response data frame creating section 54 sets, in the fourth data field 35, (i) "0x08" as the symbolic code, and (ii) "height scale Z" as the data corresponding to the symbolic code. The fourth data field 35 thus set indicates that the name of the registered instrument is "height scale Z". Moreover, the response data frame creating section 54 sets, in the fifth data field 35, (i) "0xb2" as the symbolic code and (ii) "155.6" as the data corresponding to the symbolic code. The fifth data field 35 thus set indicates that the value measured by the height scale Z is "155.6".

As described above, the user information table 3 of the vital information managing device 1 according to the present embodiment defines the effective periods of the available vital information. Therefore, the searching section 53 checks whether or not the measurement value to be transmitted is within its effective period (S73), in addition to instructing the response data frame creating section 54 to create the response data frame as described above.

Here, assume that the measurement value to be transmitted from the vital information managing device 1 according to the present embodiment was measured at 18:44 on July 2, 2006, and the present date is September 2, 2006. In this case, the present date is more than 30 days after the measurement date of the measurement value to be transmitted. As such, the present date goes beyond the vital information's effective period defined in the user information table 3, so that it is judged "NO" in S73.

In cases where the judgment in Step S73 is "NO", the searching section 53 controls the presenting section 11 such that the presenting section 11 outputs to suggest the user that she should measure the height again (S74). Examples of such outputting are (i) to display a text string such as "Please measure your height with the height scale Z again", (ii) to blink on and off the LED, and (iii) to make a warning sound from the speaker.

By thus presenting that the measurement value to be transmitted is out of the effective period, the user knows that she should measure the height with the height scale Z, before starting to carry out measurement with the weight scale X.

When presenting that the measurement value to be transmitted to the vital information measuring device 2a is out of the effective period, the response data frame creating section 54 controls the managing device communication section 13 such that the generated response data frame is transmitted to the communication address "178.23" (weight scale X) (S75). In the meanwhile, in cases where it is judged "YES" in Step S73, no particular presentation is made, and the response data frame creating section 54 controls the managing device communication section 13 such that the managing device communication section 13 transmits the created user information response data frame to the vital information measuring device 2a.

When the vital information measuring device (weight scale X) 2a receives the response data frame from the vital managing device 1 via the measuring device communication section 23, the response data frame analyzing section 63 of the vital information measuring device 2a analyzes the response data frame thus received (S55). Then, the response data frame analyzing section 63 reads out the data with reference to the device-instrument registration table 41 and the external device-instrument registration table 42 corresponding to the height scale Z. As a result of the readout, the vital information measuring device 2a is allowed to recognize that the user who wants to measure the body fat is female, was born on September 1, 1988, and has a height of 155.6 cm.

When the information necessary for the body fat measurement is acquired from the vital information managing device 1 in this way, the response data frame analyzing section 63 controls the presenting section 22 such that the presenting section 22 displays "Start measurement" or the like so as to notify the user that the vital information measuring device 2a is ready for the measurement (S56). Further, the response data frame analyzing section 63 transmits the acquired information to the measurement processing section 64.

When the presenting section 22 displays to suggest the measurement, the user moves onto a measurement position, with the result that the measurement starts. Specifically, when the user moves to the measurement position, the measurement processing section 64 calculates respective values of her weight and body fat in accordance with (i) a sensor output obtained from the measurement section 24 and (ii) the information acquired from the vital information managing device 1 (S57). Now, assume that the user's weight is 51.2 kg and the user's body fat percentage is 23% as a result of the calculation. In this case, the measurement processing section 64 causes the presenting section 22 to display the measurement results as follows: "51.2kg - 23%" (S58).

After measuring the user's weight and body fat percentage as described above, the measurement processing section 64 instructs the data frame creating section 62 to create a data frame for use in notifying the measurement results to the vital information managing device 1. In response to such an instruction, the data frame creating section 62 creates the data frame (measurement result notification data frame) for notifying thereto the respective measurement results of the weight and the body fat percentage (S59). For example, think that the above measurement results are obtained at 19:20 on July 3, 2006. In this case, the data frame creating section 62 sets the communication identifier 33 of the header portion 31 of the data frame at "Notification" and sets the service identifier 34 at "Measurement". The header portion 31 thus set indicates that the data frame is to notify the measurement results.

Further, the data frame creating section 62 sets, in the first data field 35 of the data frame portion 32, "0x01" as the symbolic code and "d" as data corresponding to the symbolic code. The first data field 35 thus set indicates information regarding the user whose internal ID in the weight scale X is "d".

The data frame creating section 62 sets, in the second data field 35, "0x02" as the symbolic code and "19:20, July 3, 2006" as data corresponding to the symbolic code. The second data field 35 thus set indicates that the measurement time is "19:20, July 3, 2006".

The data frame creating section 62 sets, in the third data field 35, "0x03" as the symbolic code and "51.2" as the data corresponding to the symbolic code. The third data field 35 thus set indicates that the measured weight is "51.2" kg.

The data frame creating section 62 sets, in the fourth data field 35, "0x04" as the symbolic code and "23" as data corresponding to the symbolic code. The fourth data field 35 thus set indicates that the measured body fat percentage is "23" %.

In this way, the data frame creating section 62 of the vital information measuring device (weight scale X) 2a uses the symbolic codes defined in the device-instrument registration table 41, so as to create the data frame for use in notifying the measurement results. Then, the data frame creating section 62 controls the measuring device communication section 23 such that the data frame thus created is transmitted to the vital information managing device 1 (S60).

When the transmission of the measurement results has been completed, the data frame creating section 62 instructs the presenting section 22 to carry out display of notifying the user that the transmission of the measurement results has been completed. In response to such an instruction from the data frame creating section 62, the presenting section 22 causes its liquid crystal display section to display a text string such as "Transmission of measurement results has been completed" for several seconds (S61). After presenting the completion of the transmission of the measurement results to the user, the vital information measuring device 2a turns off its main power source and waits for a next input from a user.

When the vital information managing device 1 receives the data frame via the managing device communication section 13, the data frame analyzing section 51 analyzes the data frame, reads out the service identifier 34 from the header portion 31, and checks it (S76). In cases where the data frame analyzing section 51 confirms that the service identifier 34 is set to indicate "Measurement", the data frame analyzing section 51 reads out each of the information contained in the data fields 35 of the data frame, and checks the measurement results of the vital information measuring device 2a with reference to the registered instrument table 5. The data frame analyzing section 51 recognizes that the information to be recorded onto the management storage section 15 are the following two information.

One of the two information is information indicating that the instrument name is "weight scale X", the internal ID is "d", the measurement value of the weight is "51.2" kg, and the measurement time is "19:20, July 3, 2006". The other one is information indicating that the instrument name is "weight scale X", the internal ID is "d", the measurement value of the body fat is "23" %, and the measurement time is "19:20, July 3, 2006".

After checking these information to be registered in the management storage section 15, the data frame analyzing section 51 notifies these information to the registering section 52, and instructs the registering section 52 to record them onto the management storage section 15. In response to such an instruction from the data frame analyzing section 51, the registering section 52 generates registration data, adds it to the vital information table 4, and records thereonto the information obtained by adding the registration data to the vital information table 4 (S77).

For improving precision in measuring vital information, the vital information communication system 100 according to the present embodiment may be configured such that: in the "user information query processing", a query is made for measurement values of the user's height in some points of time in past. That is, the measurement values in some points of time in past are taken into consideration, so that a more precise measurement result can be known.

For example, consider a case where the user asks for the following measurement values measured every one month: "18:00, May 3, 2006", "18:00 June 3, 2006", and "18:00, July 3, 2006". In this case, the frame creating section 62 creates the following data frame in Step S53 shown in Figure 15.

Specifically, the data frame creating section 62 sets the communication identifier 33 of the header portion 31 at "Request" and sets the service identifier 34 at "Inquiry". The data frame creating section 62 puts "0x01" as the symbolic code and "d" as the data corresponding to the symbolic code, in the first data field 35 of the data frame portion 32. The data frame creating section 62 puts "0x02" as the symbolic code and "18:00, May 3, 2006" as the data corresponding to the symbolic code, in the second data field 35. The data frame creating section 62 puts "0x07" as the symbolic code but the data corresponding to the symbolic code is left to be "empty", in the third data field 35. The data frame creating section 62 puts "0x02" as the symbolic code and "18:00, June 3, 2006" as the data corresponding to the symbolic code, in the fourth data field 35. The data frame creating section 62 puts "0x07" as the symbolic code but the data corresponding to the symbolic code is left to be "empty", in the fifth data field 35. The data frame creating section 62 puts "0x02" as the symbolic code and "18:00, July 3, 2006" as the data corresponding to the symbolic code, in the sixth data field 35. The data frame creating section 62 puts "0x07" as the symbolic code but the data corresponding to the symbolic code is left to be "empty", in the seventh data field 35.

As such, the data frame creating section 62 describes the data fields 35 such that the data fields in which the measurement times are contained come just before the data fields 35 regarding the queries (data fields 35 in which the data are "empty"), respectively. This allows the vital information managing device 1 to recognize that the measurement values respectively corresponding to the measurement times are being requested.

After creating the data frame having such a data structure, the data frame creating section 62 controls the measuring device communication section 23 such that the measuring device communication section 23 transmits the data frame to the vital information managing device 1 (S54).

When the vital information managing device 1 receives this data frame from the vital information measuring device (weight scale X) 2a, the data frame analyzing section 51 analyzes the data frame so as to check the instructions therein (S71). Specifically, with reference to the registered instrument table 5, the data frame analyzing section 51 recognizes that the received data frame makes queries for the measurement values of the user's height with the times designated. Then, with reference to the user measurement information table 6, the data frame analyzing section 51 confirms, in accordance with the internal ID "d" contained in the data frame, that the user ID of the user who is the measurement target is "4". The user ID "4" corresponds to the following symbolic codes representing the measurement name "height": (i) a symbolic code "0xa2" in the case of the height scale Y, and (ii) a symbolic code "0xb2" in the case of the height scale Z.

When the data frame analyzing section 51 recognizes the symbolic codes corresponding to the user ID "4" and representing the measurement name "height", the data frame analyzing section 51 instructs the searching section 53 to search for the values of the height of the user who is identified by the user ID "4", which values have been measured at the designated times respectively.

In response to such an instruction from the data frame analyzing section 51, the searching section 53 searches for measurement values whose measurement times are respectively closest to the designated times, among measurement values measured by the two height scales Y and Z in past. The search is carried out with reference to the vital information table 4.

In the present embodiment, the measurement value whose measurement time is closest to "18:00, May 3, 2006" is a measurement value "155.4" cm measured by the height scale Y at "18:44 on May 2, 2006". Further, the measurement value whose measurement time is closest to "18:00, June 3, 2006" is a measurement value "155.4" cm measured by the height scale Z at "18:30 on June 2, 2006". Further, the measurement value whose measurement time is closest to "18:00, July 3, 2006" is a measurement value "155.6" cm measured by the height scale Z at "18:44 on July 2, 2006".

In accordance with these results of the search by the searching section 53 for the measurement values respectively corresponding to the requested measurement times, the response data frame creating section 54 creates, as the user information response data frame, a data frame having the following structure (S72).

Specifically, the response data frame creating section 54 sets the communication identifier 33 of the header portion 31 at "Response" and sets the service identifier 34 at "Inquiry". The response data frame creating section 54 puts "0x01" as the symbolic code and "d" as the data corresponding to the symbolic code, in the first data field 35 of the data frame portion 32. The response data frame creating section 54 puts "0x02" as the symbolic code and "18:44, May 2, 2006" as the data corresponding to the symbolic code, in the second data field 35. The response data frame creating section 54 puts "0x07" as the symbolic code and "155.4" cm as the data corresponding to the symbolic code, in the third data field 35. The response data frame creating section 54 puts "0x02" as the symbolic code and "18:30, June 2, 2006" as the data corresponding to the symbolic code, in the fourth data field 35. The response data frame creating section 54 puts "0x07" as the symbolic code and "155.4" cm as the data corresponding to the symbolic code, in the fifth data field 35. The response data frame creating section 54 puts "0x02" as the symbolic code and "18:44, July 2, 2006" as the data corresponding to the symbolic code, in the sixth data field 35. The response data frame creating section 54 puts "0x07" as the symbolic code and "155.6" cm as the data corresponding to the symbolic code, in the seventh data field 35.

Here, assume that the present time is "18:00, July 3, 2006". In the measurement values of the user's height to be transmitted, there are measurement values whose measurement times are more than 30 days before the present time. In other words, in the measurement values to be transmitted, there are the following measurement values that are out of the effective period (30 days) set to the user whose user ID is "4": (i) the measurement value measured on May 2, 2006, and (ii) the measurement value measured on June 2, 2006.

As described above, in the vital information managing device 1 according to the present embodiment, the searching section 53 not only instructs the response data frame creating section 54 to create a response data frame, but also checks whether or not a measurement value to be transmitted is within its effective period (S73).

As a result of the check in Step S73, it is found that, in the measurement values to be transmitted to the vital information measuring device 2a, there are the measurement values that go beyond the effective period ("NO" in S73), so that the searching section 53 controls the presenting section 11 1 such that the presenting section 11 1 outputs to suggest the user remeasurement (S74).

In cases where there are such measurement values, which are out of the effective period, in the measurement values to be transmitted as described above, the presentation for instructing remeasurement is made and the response data frame created in Step S72 is transmitted to the vital information measuring device 2a (S75).

In this way, it is possible for the vital information measuring device 2a to make a query to the vital information managing device 1 for the measurement values of the user's height measured in some points of time in past. Moreover, it is possible for the vital information measuring device 2a to receive, from the vital information managing device 1, a response to the query.

As described above, the vital information managing device 2a according to the present embodiment is capable of acquiring information required for measurement processing of vital information from the managing device, thereby performing precise vital information measurement without using a storage section having a large storage space for storing information data.

Examples of the existing vital information of a general user for his/her daily health management include: weight, body fat percentage, height, the number of steps in walk, body temperature, blood pressure, and the like. Considering that healthcare business will further develop in future, the type of data used as the vital information will rapidly increase. Also, it is expected that the usage of vital information used in the medical field is to be expanded.

As the type of necessary information will increase, vast amounts of data will be required in the exchange of various types of information between devices.

As a solution of this problem, the vital information communication system 100 according to the present embodiment is configured to exchange symbolic codes each of which represents the required information. In this way, communication data amount is significantly reduced. Therefore, even if the type of vital information to be exchanged greatly increases, the required data amount will not be too large.

Further, since the vital information managing device 2 according to the present embodiment is configured to internally define the symbolic codes, there is no trouble of unifying symbolic codes for all the other vital information managing devices 2 in the vital information communication system 100.

The above-mentioned vital information managing device 1 according to the present embodiment is configured such that the searching section 53 refers to the effective period stored in the user information table 3 so as to judge whether or not the measurement time falls in the update period. This user information table 3 stores separate effective periods for the respective user IDs. The setting of the effective period is however not limited to this manner. For example, the effective periods stored in the user information table 3 may be respectively classified for the vital information managing devices 2a to 2c.

More specifically, each vital information managing device 2 may individually manage a condition(s) for its measurement operation. For example, the device-instrument registration table 41 of the vital information managing device 2 may store information regarding the effective period as a condition for the measurement operation. In the case of the device-instrument registration table 41 of Figure 3(a), the unit and the effective period value are stored as attributes to a symbolic code "0x07"(category name "height").

Then, in the "registration processing" of the device-instrument registration table 41, the information regarding the effective period is also registered to the vital information managing device 1.

Note that, the individual condition managed by each vital information managing device 2 is not limited to the effective period above, but may be (i) precision of measurement values of height, weight, etc. usable for the measurement operation by each vital information managing device 2, or (ii) a measurement condition in the measurement operation.

As described above, in the vital information communication system 100 according to the present embodiment, the presenting section 11 of the vital information managing device 1 presents an instruction of remeasurement in the case where any of the measurement values to be transmitted is out of the effective period. However, in the configuration in which the device-instrument registration table 41 of the vital information managing device 2 stores the effective period as mentioned above, the judgment as to whether the measurement value received from the vital information managing device 1 falls in (out of) the effective period may be carried out in the vital information managing device 2 with reference to the device-instrument registration table 41. In this case, if the measurement value falls out of the effective period, the presenting section 22 presents the instruction of remeasurement.

In such a configuration, as shown in Figure 1, the vital information managing device 2 further includes a judging section 65 for judging whether or not the measurement value was measured within the effective period. The judging section 65 serves similarly to the searching section 53 of the vital information managing device 1. Based on the analysis result of the response data frame analyzing section 63, the judging section 65 compares the current time with the measurement time of the received measurement value so as to judge whether or not the measurement value falls within the effective period. Further, when judging that there is a value out of the effective period, the judging section 65 causes the presenting section 22 to present the instruction of remeasurement.

Alternatively, the vital information communication system 100 may be configured as follows. The judgment as to whether the measurement value falls within the effective period is carried out in the vital information managing device 1. The judgment result is transmitted to the vital information managing device 2 together with the measurement value. Based on the judgment result, the presenting section 22 of the vital information managing device 2 presents the instruction of remeasurement. In such a configuration, the device-instrument registration table 41 to be transmitted from the vital information managing device 2 to the vital information managing device 1 and registered in the vital information managing device 1 includes additional items: e.g., symbolic code "0x09", category name "effective period", and unit "N/A". The data frames to be exchanged between the vital information managing device 2 and the vital information managing device 1 includes the followings.

First, assume that the data frame to be transmitted from the vital information managing device 2 to the vital information managing device 1 is to make a query to the vital information managing device 1 about a measurement value of height and as to whether or not the measurement value is within its effective period, for example. Such a data frame has the following data.

That is, the communication identifier 33 of the header portion 31 of the data frame is set to indicate "Request", and the service identifier 34 is set to indicate "Inquiry". The first data field 35 of the data frame portion 32 stores the symbolic code "0x01" and its corresponding data "d". The second data field 35 stores the symbolic code "0x09" but its corresponding data is left to be "empty". The third data field 35 stores the symbolic code "0x07" but its corresponding data is left to be "empty".

As such, the data field 35 immediately preceding to the data field 35 (third data field 35) that asks for the height is used for the inquiry about the effective period.

After the vital information managing device 1 receives the data frame from the vital information managing device 2a, the data frame analyzing section 51 refers to the registered instrument table 5, and analyzes the data frame. According to the analysis result, the data frame analyzing section 51 recognizes the inquiry from the vital information managing device 2 about the height measurement value and as to whether the measurement value was measured within the effective period.

More specifically, the data frame analyzing section 51 refers to the user information measurement table 6, and recognizes that the internal ID "d" corresponds to the user ID"4", and the symbolic codes "0xa2" and "0xb2" denote respective measurement names for "height" for the measurement by the height scale Y, and the measurement by the height scale Z.

Next, the searching section 53 searches for the newest measurement value among the measurement values obtained by the height scale Y and the height scale Z and stored in the vital information table 4. As a result, the searching section 53 obtains a measurement value "155.6"cm, which was measured at "18:44 on June 2, 2006".

Assume that the current time is September 2006, the searching section 53 checks the effective period corresponding to the user identified by the user ID "4", with reference to the user information table 3. Since the effective period for this user is set to 30 days, the searching section 53 judges that the measurement time of height goes beyond the effective period.

When judging as such, the searching section 53 instructs the response data frame creating section 54 such that the measurement value is to be transmitted together with the judgment result. The response data frame creating section 54 generates the following data frame, which is to be transmitted to the vital information managing device 2.

Specifically speaking, the response data frame creating section 54 sets the communication identifier 33 of the header portion 31 at "Response", and sets the service identifier 34 at "Inquiry". The first data field 35 of the data frame section 32 stores a symbolic code "0x01" and "d" as the data corresponding to the symbolic code. The second data field 35 stores a symbolic code "0x09" and "Required" as the data corresponding to the symbolic code. The third data field 35 stores a symbolic code "0x07" and "155.4" as the data corresponding to the symbolic code.

After the vital information managing device 2 receives the data frame via the measurement device communication section 23, the response data frame analyzing section 63 concludes that remeasurement is necessary according to the symbolic code "0x09". To that end, the response data frame analyzing section 63 causes the presenting section 22 to present an instruction of remeasurement of height.

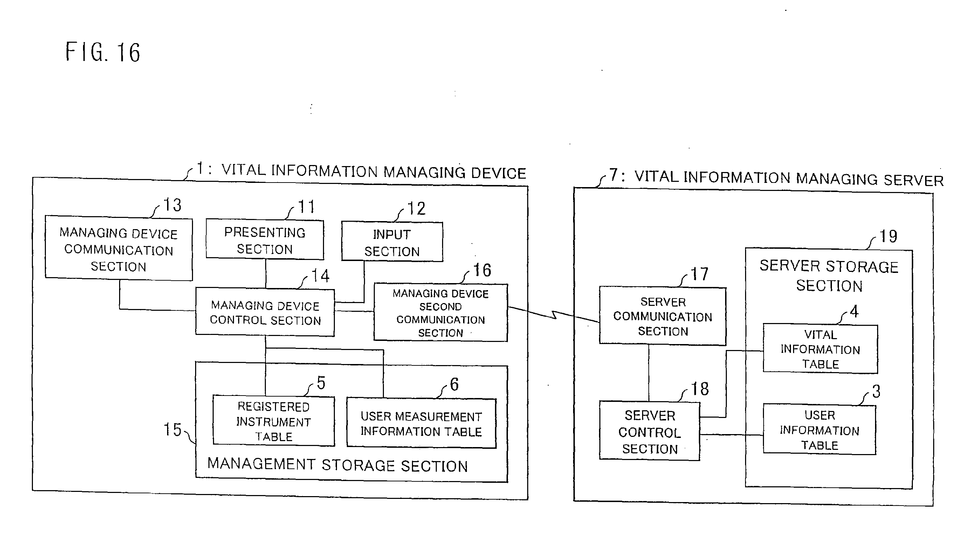

Further, as shown in Figure 16, the vital information managing device 1 according to the present embodiment may be configured to record the user information table 3 and the vital information table 4 from the information in the management storage section 15 onto the vital information managing server (server device) 7, and acquire desired information from the vital information managing server 7 as required. Figure 16 is a block diagram illustrating the configurations of main parts of a vital information managing device according to another embodiment of the present invention.

More specifically, the vital information managing device 1 of Figure 2 may be realized by a system constituted of the vital information managing device 1 and the vital information managing server 7, as shown in Figure 16.

The vital information managing device 1 of Figure 16 differs from the vital information managing device 1 of Figure 2 in that it further includes a managing device second communication section 16 (communication device) for establishing communication with the vital information managing server 7, and the management storage section 15 stores only the registered instrument table 5 and the user information measurement table 6.

On the other hand, the vital information managing server 7 serves to provide necessary information in response to requests from the vital information managing device 1. The vital information managing server 7 includes a server communication section 17 for establishing communication with the vital information managing device 1, a server control section 18 for carrying out various control over the respective sections of the vital information managing server 7, and a server storage section 19 for storing the user information table 3 and the vital information table 4.

The server control section 18 can be realized by a CPU or the like, serves to search for necessary information in the server storage section 19 in response to requests from the vital information managing device 1, and serves to cause the server communication section 17 to transmit the information thereto. The server control section 18 also serves to record information transmitted from the vital information managing device 1, onto the user information table 3 or the vital information table 4.

The registered instrument table 5 and the user information measurement table 6 can be regarded as information used for recognition of information transmitted from the vital information managing device 2, or for a search for requested information. On the other hand, the user information table 3 and the vital information table 4 can be regarded as personal information regarding the user and vital information obtained by physical measurement of the user, which are used for measurement processing or the like. Unlike that of the former, the content of the latter increases every time new data resulted from the physical measurement of user such as measurement of weight or height is inputted.

In view of this problem, as shown in Figure 16, the user information table 3 and the vital information table 4 containing such a large amount of data are stored in the vital information managing server 7 such that the vital information managing device 1 acquires the information from the user information table 3 and the vital information table 4 as required. With this configuration, the data amount in the vital information managing device 1 can be reduced. This allows the device structure of the vital information managing device 1 to be miniaturized, with the result that the user can carry the vital information managing device 1 with him/her as a mobile device.

As described above, when the measurement time of the measurement value to be transmitted to the vital information managing device 2 is out of the effective period, the presenting section 11 of the vital information managing device 1 according to the present embodiment presents an instruction of remeasurement of the value.

By configuring the vital information managing device 1 as a mobile device as described above, the user is notified of the instruction of remeasurement by such a vital information managing device 1 when the user intends to measure vital information with the vital information managing device 2 in somewhere distant from the vital information managing server 7.

That is, the instruction of measurement value remeasurement is particularly effective in the case where the user carries with him/her the vital information managing device 1 configured as shown in Figure 16.

Further, in the vital information communication system 100 according to the present embodiment, as explained in the "Registration processing" above, each vital information managing device 2 transmits the device-instrument registration table 41 to the vital information managing device 1 so as to register the device-instrument registration table 41 in the vital information managing device 1. However, instead of this, the vital information managing device 1 may be configured such that, e.g., the device-instrument registration table 41 of the vital information managing device 2 is registered therein through user's input via the input section 12. In such a configuration, the device-instrument registration table 41 inputted via the input section 12 is received by the registering section 52, and the registering section 52 records it onto the registered instrument table 5 stored in the management storage section 15.

Alternatively, in the case where the vital information managing device 1 includes a communication interface (not shown) connectable to a communication network, the vital information managing device 1 acquires the device-instrument registration table 41 of the vital information managing device 2 via the communication interface from an outer device (not shown). In this case, the registration section 52 registers the device-instrument registration table 41 in the registered instrument table 5 stored in the management storage section 15.

As described above, a vital information measuring device according to the present invention measures vital information of a living body and communicates with a managing device that manages measurement processing information necessary for measurement processing of the vital information, an identification code being assigned to an item regarding the measurement processing of the vital information, so as to specify the item, and the vital information measuring device includes: a request data generating section for generating, by using the identification code, request data for use in requesting the managing device for response data including the measurement processing data; and an analyzing section for analyzing the response data, which is received from the managing device in reply to the request data and is described by using the identification code.

This makes it possible to obtain precise vital information, even though no storage device having a particularly large storage space is provided.

As described above, a managing device according to the present invention provides, to a vital information measuring device that measures vital information of a living body, measurement processing information necessary for measurement processing carried out by the vital information measuring device, and the managing device includes: a measuring device correlation information storage device for storing measuring device correlation information indicating a correlation between an item regarding the measurement processing and an identification code, each of which is defined in the vital information measuring device; a data checking section for, when request data for requesting the measurement processing information is received from the vital information measuring device, checking the measurement processing information requested by the request data, with reference to the measuring device correlation information; a searching section for searching for the measurement processing information checked by the data checking section; and a response data generating section for generating, in accordance with the measuring device correlation information by using the identification code, response data including the measurement processing information searched by the searching section.

As such, the managing device according to the present invention provides the measurement processing information to the vital information measuring device, which includes no storage device having a particularly large storage space, with the result that the vital information measuring device can obtain precise vital information.

As described above, a vital information communication system according to the present invention includes: a vital information measuring device, which measures vital information of a living body and communicates with a managing device that manages measurement processing information necessary for measurement processing of the vital information, an identification code being assigned to an item regarding the measurement processing of the vital information, so as to specify the item, said vital information measuring device including (i) a request data generating section for generating, by using the identification code, request data for use in requesting the managing device for response data including the measurement processing data and (ii) an analyzing section for analyzing the response data, which is received from the managing device in reply to the request data and is described by using the identification code; and a managing device, which provides, to the vital information measuring device that measures the vital information of the living body, the measurement processing information necessary for the measurement processing carried out by the vital information measuring device, the managing device including (i) a measuring device correlation information storage device for storing measuring device correlation information indicating a correlation between the item regarding the measurement processing and the identification code, each of which is defined in the vital information measuring device, (ii) a data checking section for, when the request data for requesting the measurement processing information is received from the vital information measuring device, checking the measurement processing information requested by the request data, with reference to the measuring device correlation information, (iii) a searching section for searching for the measurement processing information checked by the data checking section, and (iv) a response data generating section for generating, in accordance with the measuring device correlation information by using the identification code, the response data including the measurement processing information searched by the searching section.

Accordingly, even though the vital information measuring device is not provided with a storage device having a particularly large storage space, the vital information measuring device can obtain precise vital information.

As described above, a method according to the present invention for controlling a vital information measuring device, which measures vital information of a living body and communicates with a managing device that manages measurement processing information necessary for measurement processing of the vital information, an identification code being assigned to an item regarding the measurement processing of the vital information, so as to specify the item, includes: a step of generating, by using the identification code, request data for use in requesting the managing device for response data including the measurement processing data; and a step of analyzing the response data, which is received from the managing device in reply to the request data and is described by using the identification code.

This makes it possible to obtain precise vital information, even though no storage device having a particularly large storage space is provided.

As described above, a method according to the present invention for controlling a managing device, which provides, to a vital information measuring device that measures vital information of a living body, measurement processing information necessary for measurement processing carried out by the vital information measuring device, includes: a step of storing measuring device correlation information indicating a correlation between an item regarding the measurement processing and an identification code, each of which is defined in the vital information measuring device; a step of, when request data for requesting the measurement processing information is received from the vital information measuring device, checking the measurement processing information requested by the request data, with reference to the measuring device correlation information; a step of searching for the measurement processing information checked by the data checking section; and a step of generating, in accordance with the measuring device correlation information by using the identification code, response data including the measurement processing information searched by the searching section.

Accordingly, the method for controlling the managing device according to the present invention makes it possible to provide the measurement processing information to the vital information measuring device, which does not include a storage device having a particularly large storage space, with the result that the vital information measuring device can obtain precise vital information.

Further, it is preferable that the above-arranged vital information measuring device according to the present invention further include an external device correlation information storage device for storing external device correlation information indicating a correlation between the item regarding the measurement processing and an identification code, each of which is defined in an external vital information measuring device, wherein: in cases where the response data includes information indicated by the identification code defined by the external vital information measuring device, the analyzing section analyzes the response data with reference to the external device correlation information stored in the external device correlation information storage device.

According to the above structure, the external correlation information storage section is provided, so that it is possible to analyze the received response data with reference to the external device correlation information, even if the response data has a part described by using the identification code defined in the external vital information measuring device.

In other words, the vital information measuring device according to the present invention is capable of handling information described by using the identification code defined in the external vital information measuring device.

Further, the above-arranged vital information measuring device may be arranged such that: in cases where the response data includes device information indicating the external vital information measuring device, which has provided the measurement processing information that is being managed by the managing device, the analyzing section checks the device information of the response data and records, onto the external device correlation information storage device, existence information indicating whether or not there exists the measurement processing information having been provided by the external information measuring device.

According to the above structure, in cases where the response data includes the device information, the analyzing section can check the device information, so that it is possible to check the existence of the measurement processing information having been provided by the external vital information measuring device. Further, the result of this check can be recorded as the existence information, so that the vital information measuring device according to the present invention can check for usable measurement processing information having been provided by external vital information measuring devices.

Further, it is preferable that the above-arranged vital information measuring device further include: a calculating section for calculating, in accordance with a result of the measurement of the vital information of the living body, vital information, wherein: in cases where the response data includes, as the measurement processing information, a measurement value required in calculating the vital information and measured by an external vital information measuring device, the calculating section calculates the vital information in accordance with (i) the result of the measurement and (ii) the measurement value included in the response data and measured by the external information measuring device.

According to the above structure, the calculating section is provided, so that it is possible to calculate the measurement value of the vital information, which measurement value cannot be obtained by merely measuring the vital information of the living body.

It is preferable to arrange the above-arranged vital information measuring device according to the present invention such that: each of the request data and the response data includes (i) a header describing a purpose of processing requested to each of the devices to which the request data and the response data are transmitted respectively, and (ii) a data frame including a combination of an item of information to be transmitted and a value corresponding to the item of the information.

Further, the above-arranged vital information measuring device according to the present invention may be arranged such that: in cases where the value corresponding to the item is requested to the managing device, the requested value corresponding to the item is not described in the data frame of the request data.

Further, it is preferable that, in the above-arranged vital information measuring device according to the present invention, an effective period indicating a period during which the measurement processing information is usable is set in the measurement processing information, and that the vital information measuring device further include: a judging section for judging whether or not the measurement processing information is within the effective period, in cases where the response data received from the managing device includes the measurement processing information; and a measuring device output device for outputting information of instructing update of the measurement processing information, when the judging section judges that the measurement processing information is out of the effective period.

According to the above structure, the judging section is provided, so that it is possible to judge whether or not the measurement processing information is out of the effective period. Moreover, the measuring device output device is provided. Therefore, when the judging section judges that the measurement processing information is out of the effective period, it is possible to output such information as to instruct update of the measurement processing information. This induces updating of the measurement processing information to more reliable information.

The above-arranged managing device according to the present invention may further include: a measurement processing information storage device for storing the measurement processing information, wherein: the searching section searches, in the measurement processing information storage device, for the measurement processing information checked by the data checking section.

Further, the above-arranged managing device according to the present invention may be arranged such that: the measurement processing information includes (i) measurement target person information regarding a measurement target person, and (ii) measurement result information regarding measurement results received from vital information measuring devices, and the measurement processing information storage device includes (i) a measurement target person information storage device for storing the measurement target person information, and (ii) a measurement result information storage device for storing the measurement result information.

The above-arranged managing device according to the present invention may further include: a communication device for establishing communication with a server device storing the measurement processing information, wherein: via the communication device, the searching section searches for the measurement processing information in the server device and acquires the measurement processing information from the server device.

Examples of the measurement processing information include: (i) a measurement result measured by an external vital information measuring device, (ii) information regarding a measurement target person, and the like. The data amount of the measurement processing information increases as the number of managed measurement results and the number of measurement target persons are increased.

According to the above structure, only the measurement processing information, whose data amount increases as the aforesaid numbers are increased, is stored in the server device. Required measurement processing information is acquired from the server device in response to a request from the vital information measuring device.

Hence, the managing device according to the present invention does not need to have a storage device having a particularly large storage space, and can be therefore miniaturized, so that the measurement target person can bring the managing device with him/her.

Further, it is preferable that the above-arranged managing device according to the present invention further include: a managing device output device for outputting information for a measurement target person, wherein: an effective period indicating a period during which the measurement processing information is usable is set in the measurement processing information, the searching section judges whether or not the measurement processing information thus searched is within the effective period, and in cases where the searching section judges that the measurement processing information is out of the effective period, the searching section causes the managing device output device to output information of instructing update of the measurement processing information.

According to the above structure, when the searching section judges that the measurement processing information is out of the effective period, it is possible to output such information as to instruct update of the measurement processing information. This induces updating of the measurement processing information to more reliable information.

Further, the above-arranged managing device according to the present invention may further include: a registering section for registering information received from the vital information measuring device, wherein: in cases where the measuring device correlation information defined in the vital information measuring device is received from the vital information measuring device, the registering section registers the measuring device correlation information in the measuring device correlation information storage device such that the measuring device correlation information is correlated with device specifying information for specifying the vital information measuring device.

According to the above structure, the registering section is provided, so that it is possible to register and manage the measuring device correlation information for each vital information measuring device, in the measuring device correlation information storage device.

Further, it is preferable that the managing device according to the present invention further include: a registering section for registering information received from the vital information measuring device, wherein: in cases where the vital information measured by the vital information measuring device is received from the vital information measuring device, the registering section registers the vital information in the measurement processing information storage device as the measurement processing information.

According to the above structure, the managing device receives, from the vital information measuring device, the vital information measured by the vital information measuring device, and registers the vital information in the measurement processing information storage device. This makes it possible that the measurement processing information includes the newest information.

Note that each of the vital information measuring device and the managing device may be realized by a computer. In this case, the present invention encompasses (i) a program for controlling the vital information measuring device, (ii) a program for controlling the managing device, and (iii) computer-readable storage mediums respectively storing the programs. The program for controlling the vital information measuring device realizes the vital information measuring device by the-computer by causing the computer to operate as the above sections of the vital information measuring device. The program for controlling the managing device realizes the managing device by the computer by causing the computer to operate as the above sections of the managing device.

The present invention is not limited to the description of the embodiments above, but may be altered by a skilled person within the scope of the claims. An embodiment based on a proper combination of technical means disclosed in different embodiments is encompassed in the technical scope of the present invention.

Finally, each of the blocks of the vital information measuring device 2 and the vital information managing device 1, especially the request checking section 61, the data frame creating section 62, the response data frame analyzing section 63, the measurement processing section 64, the data frame analyzing section 51, the registering section 52, the searching section 53, and the response data frame creating section 54 may be constituted by hardware logic, or may be realized by software with the use of a CPU as follows.

Specifically, each of the vital information measuring device 2 and the vital information managing device 1 includes: (i) a CPU (central processing unit) for executing instructions of a control program realizing each function; (ii) a ROM (read only memory) storing the above program; (iii) a RAM (random access memory) for expanding the program; (iv) a storage device (storage medium), such as a memory, storing the program and various types of data; and the like. Therefore, the object of the present invention is achieved by: (i) providing, in the vital information measuring device 2 or the vital information managing device 1, a storage medium which stores a computer-readable program code (executable program, intermediate code program, a source program) of the control program of the vital information measuring device 2 or the vital information managing device 1 each of which is software for realizing the function, and (ii) causing the computer (CPU, or MPU) to read out and execute the program code stored in the storage medium.

Examples of the storage medium are: tapes such as a magnetic tape and a cassette tape; magnetic disks such as a floppy® disk and a hard disk; disks such as a CD-ROM (compact disk read only memory), a magnetic optical disk (MO), a mini disk (MD), a digital video disk (DVD), and a CD-Recordable (CD-R); and the like. Further, the storage medium may be: a card such as an IC card or an optical card; or a semiconductor memory such as a mask ROM, an EPROM (electrically programmable read only memory), EEPROM (electrically erasable programmable read only memory), or a flash ROM.

Further, the vital information measuring device 2 or the vital information managing device 1 may be so arranged as to be connectable to a communication network, and the program code may be supplied thereto via the network. The communication network is not particularly limited. Specific examples thereof are: the Internet, intranet, extranet, LAN (local area network), ISDN (integrated services digital network), VAN (value added network), CATV (cable TV) communication network, virtual private network, telephone network, mobile communication network, satellite communication network, and the like. Further, a transmission medium (channel) constituting the communication network is not particularly limited. Specific examples thereof are: (i) a wired channel using an IEEE1394, a USB (universal serial bus), a power-line communication, a cable TV line, a telephone line, a ADSL line, or the like; or (ii) a wireless channel using IrDA, infrared rays used for a remote controller, Bluetooth®, IEEE802.11, HDR (High Data Rate), a mobile phone network, a satellite connection, a terrestrial digital network, or the like. Note that the present invention can be realized by a form of a computer data signal embedded in a carrier wave realized by electronic transmission of the program code.

### [INDUSTRIAL APPLICABILITY]

The vital information measuring device 2 according to the present embodiment includes the data frame creating section 62 and the response data frame analyzing section 63. Therefore, by using the data frame described by the symbolic code, the vital information measuring device 2 makes a request to the vital information managing device 1 for the information required in the measurement processing. Accordingly, the present invention is effectively applicable in cases where information obtained by external devices are shared by devices having small usable resources. An example of such devices having small usable resources is the vital information measuring device 2 according to the present embodiment.

## Claims

1. A vital information measuring device (2), which measures vital information of a living body and communicates with a managing device (1) that manages measurement processing information necessary for measurement processing of the vital information,
said vital information measuring device (2), **characterized in that** an identification code is assigned to an item regarding the measurement processing of the vital information, so as to specify the item,
said vital information measuring device (2), **characterized by** comprising:
a request data generating section (62) for generating, by using the identification code, request data for use in requesting the managing device (1) for response data including the measurement processing data; and
an analyzing section (63) for analyzing the response data, which is received from the managing device (1) in reply to the request data and is described by using the identification code.

2. The vital information measuring device (2) as set forth in claim 1, **characterized by** further comprising:
an external device correlation information storage device (26) for storing external device correlation information (42) indicating a correlation between the item regarding the measurement processing and an identification code, each of which is defined in an external vital information measuring device,
**characterized in that**:
in cases where the response data includes information indicated by the identification code defined by the external vital information measuring device, the analyzing section (63) analyzes the response data with reference to the external device correlation information (42) stored in the external device correlation information storage device (26).

3. The vital information measuring device (2) as set forth in claim 2, **characterized in that**:
in cases where the response data includes device information indicating the external vital information measuring device, which has provided the measurement processing information (3, 4) that is being managed by the managing device, the analyzing section (63) checks the device information of the response data and records, onto the external device correlation information storage device (26), existence information (43) indicating whether or not there exists the measurement processing information (3, 4) having been provided by the external information measuring device.

4. The vital information measuring device (2) as set forth in any one of claims 1 to 3, **characterized by** further comprising:
a calculating section (64) for calculating, in accordance with a result of the measurement of the vital information of the living body, vital information,
**characterized in that**:
in cases where the response data includes, as the measurement processing information, a measurement value required in calculating the vital information and measured by an external vital information measuring device, the calculating section (64) calculates the vital information in accordance with (i) the result of the measurement and (ii) the measurement value included in the response data and measured by the external information measuring device.

5. The vital information measuring device (2) as set forth in any one of claims 1 to 4, **characterized in that**:
each of the request data and the response data includes (i) a header (31) describing a purpose of processing requested to each of the devices to which the request data and the response data are transmitted respectively, and (ii) a data frame (32) including a combination of an item of information to be transmitted and a value corresponding to the item of the information.

6. The vital information measuring device (2) as set forth in claim 5, **characterized in that**:
in cases where the value corresponding to the item is requested to the managing device (1), the requested value corresponding to the item is not described in the data frame (3) of the request data.

7. The vital information measuring device (2) as set forth in any one of claims 1 to 6, in which an effective period indicating a period during which the measurement processing information is usable is set in the measurement processing information (3, 4),
said vital information measuring device (2), **characterized by** further comprising:
a judging section (65) for judging whether or not the measurement processing information (3, 4) is within the effective period, in cases where the response data received from the managing device (1) includes the measurement processing information (3, 4); and
a measuring device output device (22) for outputting information of instructing update of the measurement processing information (3, 4), when the judging section (65) judges that the measurement processing information (3, 4) is out of the effective period.

8. A managing device (1), which provides, to a vital information measuring device (2) that measures vital information of a living body, measurement processing information (3, 4) necessary for measurement processing carried out by the vital information measuring device (2),
said managing device (1), **characterized by** comprising:
a measuring device correlation information storage device (15) for storing measuring device correlation information (5) indicating a correlation between an item regarding the measurement processing and an identification code, each of which is defined in the vital information measuring device (2);
a data checking section (51) for, when request data for requesting the measurement processing information is received from the vital information measuring device (2), checking the measurement processing information requested by the request data, with reference to the measuring device correlation information (5);
a searching section (53) for searching for the measurement processing information checked by the data checking section (51); and
a response data generating section (54) for generating, in accordance with the measuring device correlation information (5) by using the identification code, response data including the measurement processing information searched by the searching section (53).

9. The managing device (1) as set forth in claim 8, **characterized by** further comprising:
a measurement processing information storage device (15) for storing the measurement processing information,
**characterized in that**:
the searching section (53) searches, in the measurement processing information storage device (15), for the measurement processing information checked by the data checking section (51).

10. The managing device (1) as set forth in claim 9, **characterized in that**:
the measurement processing information (3, 4) includes (i) measurement target person information (3) regarding a measurement target person, and (ii) measurement result information (4) regarding measurement results received from vital information measuring devices, and
the measurement processing information storage device (15) includes (i) a measurement target person information storage device for storing the measurement target person information, and (ii) a measurement result information storage device for storing the measurement result information.

11. The managing device (1) as set forth in claim 8, **characterized by** further comprising:
a communication device (16) for establishing communication with a server device (7) storing the measurement processing information (3, 4),
**characterized in that**:
via the communication device (16), the searching section (53) searches for the measurement processing information in the server device (7) and acquires the measurement processing information (3, 4) from the server device (7).

12. The managing device (1) as set forth in any one of claims 8 to 11, **characterized by** further comprising:
a managing device output device (11) for outputting information for a measurement target person,
**characterized in that**:
an effective period indicating a period during which the measurement processing information (3, 4) is usable is set in the measurement processing information (3, 4),
the searching section (53) judges whether or not the measurement processing information (3, 4) thus searched is within the effective period, and
in cases where the searching section (53) judges that the measurement processing information is out of the effective period, the searching section (53) causes the managing device output device (11) to output information of instructing update of the measurement processing information (3, 4).

13. The managing device (1) as set forth in any one of claims 8 to 12, **characterized by** further comprising:
a registering section (52) for registering information received from the vital information measuring device (2),
**characterized in that**:
in cases where the measuring device correlation information (5) defined in the vital information measuring device (2) is received from the vital information measuring device (2), the registering section (52) registers the measuring device correlation information (5) in the measuring device correlation information storage device (15) such that the measuring device correlation information (5) is correlated with device specifying information for specifying the vital information measuring device.

14. The managing device (1) as set forth in any one of claims 9 to 12, further comprising:
a registering section (52) for registering information received from the vital information measuring device (2),
**characterized in that**:
in cases where the vital information measured by the vital information measuring device (2) is received from the vital information measuring device (2), the registering section (52) registers the vital information in the measurement processing information storage device (15) as the measurement processing information.

15. A vital information communication system, **characterized by** comprising:
the vital information measuring device as set forth in any one of claims 1 to 7; and
the managing device as set forth in any one of claims 8 to 14.

16. A method for controlling a vital information measuring device (2), which measures vital information of a living body and communicates with a managing device (1) that manages measurement processing information necessary for measurement processing of the vital information,
said method, **characterized in that**:
an identification code being assigned to an item regarding the measurement processing of the vital information, so as to specify the item,
said method, **characterized by** comprising:
a step (S33, S53) of generating, by using the identification code, request data for use in requesting the managing device (1) for response data including the measurement processing data (3, 4); and
a step (S35, S55) of analyzing the response data, which is received from the managing device (1) in reply to the request data and is described by using the identification code.

17. A method for controlling a managing device (1), which provides, to a vital information measuring device (2) that measures vital information of a living body, measurement processing information (3, 4) necessary for measurement processing carried out by the vital information measuring device (2),
said method, **characterized by** comprising:
a step (S22) of storing measuring device correlation information (5) indicating a correlation between an item regarding the measurement processing and an identification code, each of which is defined in the vital information measuring device (2);
a step (S71) of, when request data for requesting the measurement processing information (3, 4) is received from the vital information measuring device (2), checking the measurement processing information requested by the request data, with reference to the measuring device correlation information (5);
a step (S72) of searching for the measurement processing information (3, 4) checked; and
a step (S72) of generating, in accordance with the measuring device correlation information (5) by using the identification code, response data including the measurement processing information (3, 4) searched.

18. A control program for causing the vital information measuring device (2) as set forth in any one of claims 1 to 7 to operate, said control program causing a computer to function as the respective sections.

19. A control program for causing the managing device (1) as set forth in any one of 8 to 14 to operate, said control program causing a computer to function as the respective sections.

20. A computer-readable storage medium, storing the control program, as set forth in claim 18, of the vital information measuring device (2).

21. A computer-readable storage medium, storing the control program, as set forth in claim 19, of the managing device (1).
